# EUROPEAN PATENT SPECIFICATION

(11) **EP 3 412 766 B1**
(45) Date of publication and mention of the grant of the patent: **27.01.2021**
(21) Application number: 17382340.2
(22) Date of filing: 06.06.2017
(51) Int. Cl.: C12N 1/20, C12R 1/225

(54) **LACTOBACILLUS SALIVARIUS STRAIN, COMPOSITION COMPRISING THE SAME, AND USES THEREOF**
LACTOBACILLUS SALIVARIUS STAMM, ZUSAMMENSETZUNG, DIE DER STAMM BEINHALTEN, UND VERWENDUNG DAVON
SOUCHE DE LACTOBACILLUS SALIVARIUS, COMPOSITION COMPRENANT LES MÊMES ET LEURS UTILISATIONS

(43) Date of publication of application: 12.12.2018
(73) Proprietor: Casen Recordati, S.L., 50180 Utebo (Zaragoza) (ES)
(72) Inventor: BELLO NAVARRO, Marta, 50180 Utebo (ES); RODRÍGUEZ GÓMEZ, Juan Miguel, 28770 Colmenar Viejo (ES); CÁRDENAS CÁRDENAS, Nivia, 28053 Madrid (ES); MARTÍN MERINO, Virginia, 28007 Madrid (ES)
(74) Representative: ZBM Patents - Zea, Barlocci & Markvardsen

(56) References cited:
- EP-A1- 3 000 473
- WO-A1-03/010298

### SEQUENCE LISTING

<110> CASEN RECORDATI, S.L.
<120> Lactobacillus salivarius strain, composition comprising the same, and uses thereof
<130> P3680EP00
<160> 5
<170> PatentIn version 3.5
<210> 1
   <211> 20
   <212> DNA
   <213> Artificial Sequence
<220>
   <223> Primer pb116
<400> 1
   agagtttgat cctggctcag 20
<210> 2
   <211> 20
   <212> DNA
   <213> Artificial Sequence
<220>
   <223> Primer mb116
<400> 2
   ggctgctggc acgtagttag 20
<210> 3
   <211> 10
   <212> DNA
   <213> Artificial Sequence
<220>
   <223> Primer OPL5
<400> 3
   acgcaggcac 10
<210> 4
   <211> 19
   <212> DNA
   <213> Artificial Sequence
<220>
   <223> Primer S.agalacFW1
<400> 4
   gtttggtgtt tacactaga 19
<210> 5
   <211> 22
   <212> DNA
   <213> Artificial Sequence
<220>
   <223> Primer S.agalacRV1
<400> 5
   caattgctcc ttttaaataa ct 22

## Description

### Technical Field

The present invention provides a new isolated strain of the microorganism *Lactobacillus salivarius,* compositions comprising it and uses thereof in the prevention and/or treatment of infection diseases, particularly of infection diseases caused by *Streptococcus agalactiae,* more particularly of neonatal sepsis, gynecological infection and/or mastitis.

### Background art

Neonatal sepsis contributes substantially to neonatal morbidity and mortality, and is a major global public health challenge worldwide. According to the onset of age, neonatal sepsis is divided into early-onset sepsis (EOS) and late-onset sepsis (LOS). EOS has been variably defined based on the age at onset, with bacteremia or bacterial meningitis occurring at ≤72 h in infants hospitalized in the neonatal intensive care unit versus <7 days in term infants. EOS reflects transplacental or, more frequently, ascending infections from the maternal genitourinary tract, whereas LOS is associated with the postnatal nosocomial or community environment, with a peak incidence reported to be between days 10 and 22 after birth (Van den Hoogen *et al*., 2010).

One of the microorganisms most frequently involved in EOS, taking into account both term and preterm infants together, is *S*. *agalactiae* (group B streptococci, GBS). Women, men and children of all ages in both developed and developing countries can be colonized with GBS without having any symptoms. The gastrointestinal tract, vagina and urethra serve as reservoirs for GBS. In addition to asymptomatic carriage, GBS can also cause maternal infections, including urinary tract infections, vulvovaginitis, cervical dysplasia, endometritis, intra-amniotic infection and wound infections (Muller, *et al*., 2006; Savini *et al*., 2013). The maternal colonization rate is, approximately, 18% (ranging from 12 to 28%), independently of the countries or socio-economic status of the screened women. Neonates with GBS neonatal infection, either as EOS or as LOS, may present with respiratory disease (54%), sepsis without focus (27%) and/or meningitis (15%).

The most commonly used prevention for GBS infection is intrapartum chemoprophylaxis (intrapartum antibiotic prophylaxis, IAP) with antibiotics to mothers with known GBS colonization or unknown status. In relation to IAP, the biggest concerns are the increasing rates of antibiotic resistance among clinically relevant microorganisms and the impact on the acquisition, composition and development of the infant microbiota.

Another strategy against GBS has been the search of a vaccine. However, the known vaccines do not confer protection against all GBS serotypes. Even if an effective vaccine to prevent GBS infection is developed in the future, a need for an alternative is necessary as all women will not be immunized and the vaccine may not be effective in women giving birth preterm.

Some studies have evaluated the potential of different lactic acid bacteria strains or their metabolites to inhibit the growth of *S*. *agalactiae* (De Gregorio *et al*., 2014). Even that efforts have been made, results *in vitro* do not correlate with results *in vivo* in urogenital tract infections (De Gregorio *et al*., 2014), pointing out the importance of human clinical assays in the efforts to eradicate *S*. *agalactiae* during pregnancy.

GBS is also responsible of mastitis (inflammation of the udder) in ruminants, such as cattle, and causes decrease in the quantity and quality of milk products produced. Moreover, cattle can be a reservoir of *S*. *agalactiae* strains that could cause disease and death in infants. GBS is also responsible of mastitis in humans.

Therefore, there is a need for efficient prevention of colonization by GBS, of prevention of infections caused by said bacteria in mammals and for therapeutic approaches in the treatment of diseases caused by said microorganism such as neonatal sepsis.

### Summary of the invention

The inventors have found one strain of *L. salivarius* that antagonizes with GBS and prevents GBS infection *in vivo.*

The strain of the invention is a probiotic safe strain with the *in vitro* and *in vivo* ability to eradicate GBS from the intestinal and genitourinary tracts of pregnant women and/or their infants.

The inventors have further found that the identified strain (a) produced high amounts of lactic acid, (b) was able to produce peroxide hydrogen; and (c) when compared to other strains, it was found that the strain of the invention showed the best combination of adherence to epithelial cells, co-aggregation with GBS and inhibition of GBS strains in broth co-cultures.

In a clinical trial focused on the rectal and vaginal eradication of GBS in pregnant women by using the probiotic strain of the present invention it was found that the strain of the invention allowed a notable reduction in the rate of GBS-colonized women and led to a sharp reduction in the use of antibiotics during the peripartum period.

Thus, the first aspect of the present invention refers to an isolated strain of *L. salivarius* deposited at the "Colección Española de Cultivos Tipo (CECT)" under the accession number CECT 9145. This strain is also named as "the strain of the invention".

The strain of *Lactobacillus salivarius* V4II-90 was isolated from a vaginal exudate of a woman in Spain and was deposited, according to the Budapest Treaty, at the "Colección Española de Cultivos Tipo (CECT)" in the Universidad de Valencia C.P 46980 Catedrático Agustin Escardino N° 9 Paterna, Valencia (Spain), under the accession number CECT 9145. It was deposited by the depositor Casen Recordati, S.L. (Autovía de Logroño, km 13,300, 50180 Utebo, Zaragoza, Spain) on the 26 April 2016. The strain was identified by the depositor with the reference V4II-90, and received the accession number CECT 9145. It was, in addition, declared viable.

In the present invention the terms V4II-90 and CECT 9145 are used interchangeably.

A second aspect of the present invention refers to a bacterial culture which comprises the strain of the first aspect of the invention. The bacterial culture can be a bacterial pure culture.

A third aspect of the present invention refers to the isolated strain according to the first aspect of the invention or the bacterial culture of the second aspect of the invention for use as a medicament.

A fourth aspect of the present invention refers to the isolated strain of the first aspect of the invention or the bacterial pure culture of the second aspect of the invention for use in the prevention and/or treatment of an infection disease. The inventors have found that the strain of the invention has antimicrobial activity against *S*. *agalactiae* strains: *in vitro* against strains from newborns with neonatal sepsis and against strains from pregnant women; and also *in vivo* in pregnant women.

As will be illustrated in the examples below, the strain of the first aspect of the invention can also be used as a probiotic according to the FAO guidelines, which are the guidelines emitted by Food and Agriculture Organization of the United Nations.

The strain of the invention is safe, survives after transit through the gastrointestinal tract and can be isolated after oral intake in the intestine and vaginal tracks in pregnant women.

Therefore a fifth aspect of the present invention refers to the use of the isolated strain of the first aspect of the invention or the bacterial pure culture of the second aspect of the invention as a probiotic, a nutraceutical product, a food preparation or a food supplement.

The strain of the invention can be supplied as an ingredient of a composition comprising carriers or other ingredients. Therefore, a sixth aspect of the present invention refers to a pharmaceutical composition comprising a therapeutically effective amount of the strain of the first aspect of the invention or the bacterial culture of the second aspect of the invention as active ingredient, together with appropriate amounts of pharmaceutical acceptable excipients and/or carriers. As it is illustrated in the examples, the strain *L. salivarius* CECT 9145 is a safe strain displaying probiotic properties. For this reason, it can be administered in the form of an edible product which comprises an effective amount of the strain of the invention together with appropriate amounts of other edible ingredients (i.e. compounds that can be safely eaten).

Therefore, a seventh aspect of the present invention is an edible product which comprises an effective amount of the strain of the first aspect of the invention (*L*. *salivarius* CECT 9145) or a bacterial culture of the second aspect of the invention as active ingredient, together with appropriate amounts of other edible ingredients.

It is known by the expert of the field that Lactobacilli can be used as vaccine delivery vectors or as adjuvants. Therefore another aspect of the present invention refers to the use of the strain of the first aspect of the invention, or the bacterial culture of the second aspect of the invention, or the pharmaceutical composition of the sixth aspect of the present invention as vaccine delivery vector or alternatively as adjuvant.

An eighth aspect of the present invention relates to a medical device comprising an effective amount of the strain of the first aspect of the invention, the bacterial culture of the second aspect of the invention or the pharmaceutical composition of the sixth aspect of the invention as an active ingredient.

A ninth aspect of the present invention relates to a method to obtain a mutant or a variant thereof of the strain of *L. salivarius* CECT 9145, comprising using the deposited strain as defined in the first aspect of the invention as starting material and applying mutagenesis, wherein the obtained mutant retains or enhances the activity of the parent deposited strain to prevent or treat an infection disease, particularly an infection disease caused by *S*. *agalactiae.*

A tenth aspect of the present invention relates to a kit that comprises an effective amount of the strain of the first aspect of the invention, of the bacterial culture of the second aspect of the invention or of the pharmaceutical composition of the sixth aspect of the invention.

A particular embodiment of the invention refers to the use of the isolated strain, the bacterial culture, the pharmaceutical composition or the edible product of the present invention in the prevention and/or treatment of an infection disease, in a particular embodiment, the infection disease caused by *S. agalactiae.*

### Brief description of the drawings

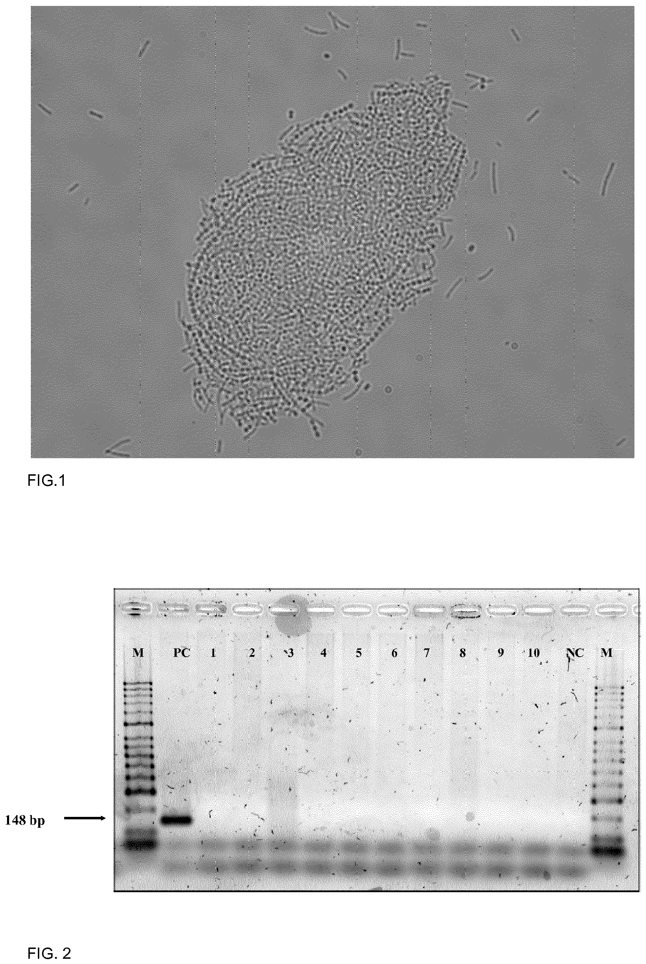

FIG.1. Shows the strong co-aggregation between *L. salivarius* V4II-90 and a *S.*
*agalactiae* strain.
FIG.2. Electrophoretic analysis of PCR product of the 148 bp region of *16S rRNA gene* with the use of primers *S.agalacFW1* and *S.agalacRV1* obtained from *Streptococcus peroris* DSM 12493 (lane 1); *Streptococcus mitis* DSM 12643 (lane 2); *Streptococcus oralis* CECT 907 (lane 3); *Streptococcus parasanguinis* DSM 6778 (lane 4); *Streptococcus bovis* DSM 20564 (lane 5); *Streptococcus uberis* DSM 20564 (lane 6); *Streptococcus salivarius* CECT 805 (lane 7); *Streptococcus thermophilus,* ATCC 19987 (lane 8); *Streptococcus pneumoniae* 0566 (lane 9); *Streptococcus dysgalactiae* DSM 20662 (lane 10). "M", Molecular weight marker HyperLader™ IV (Bioline); "PC", positive control, S. *agalactiae,* DSMZ 2134; "NC", negative control. This figure is a reverse image of the agarose gels containing Gel Red™ Nucleic Acid gel Stain 10.000X (Biotium). "bp", base pair.

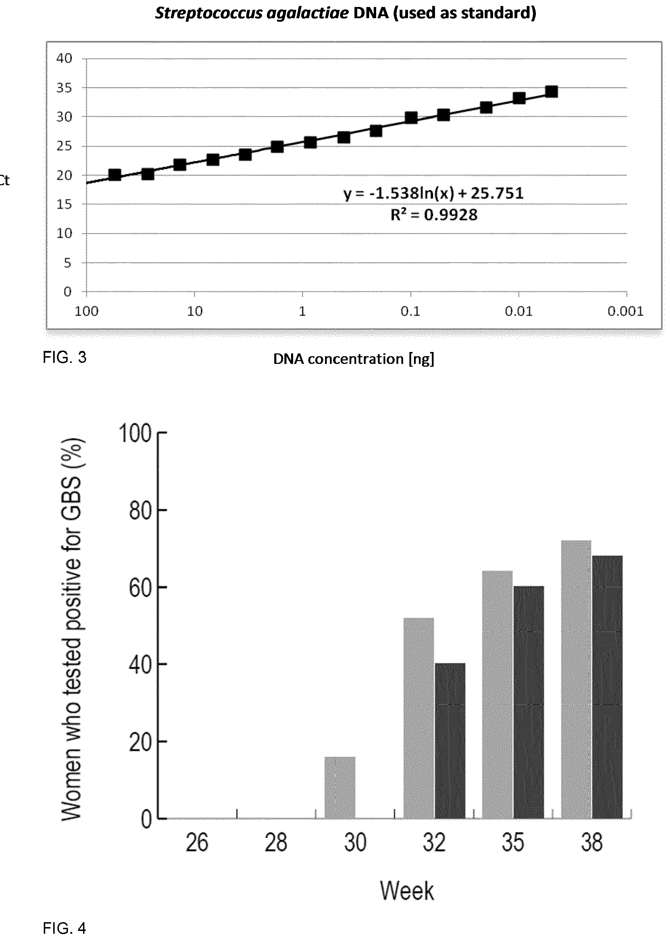

FIG. 3. Detection limit of the real-time PCR system, using ten-fold dilution series of S. *agalactiae* DSMZ 2134 DNA as standard.
FIG. 4. Qualitative assessment of *S. agalactiae* (GBS) in rectal (grey bars) and vaginal (black bars) swabs taken regularly from participants who initially tested positive for GBS and received oral administration of *L. salivarius* V4II-90 (10⁹ cfu/daily) (n = 25). "cfu", colony formation unit.

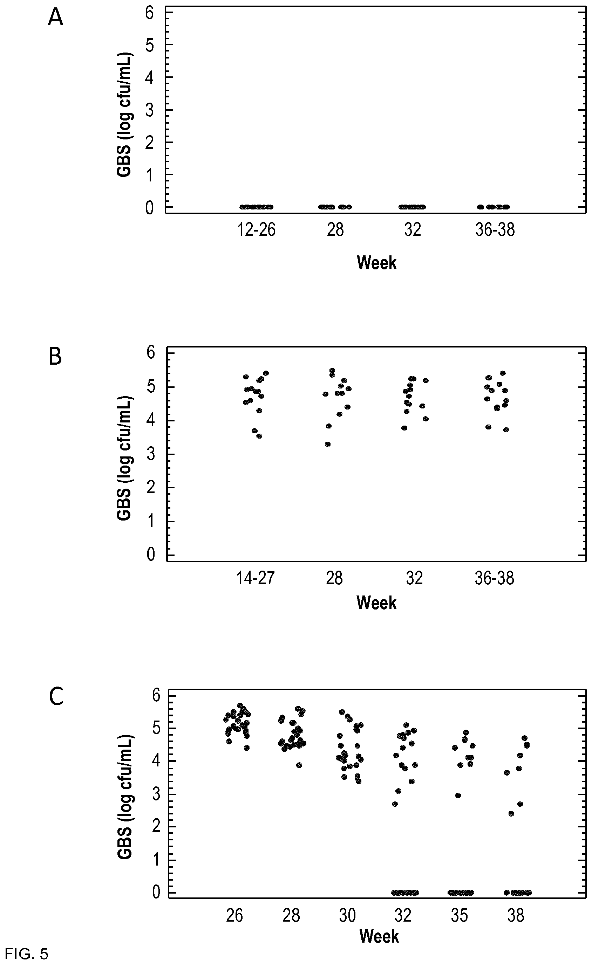

FIG. 5. Concentration (CFU/mL) of *S. agalactiae* (GBS) in vaginal swabs taken regularly from A) GBS-negative women (n = 18) without oral administration of *L. salivarius* V4II-90 (10⁹ cfu/daily); B) GBS-positive women (n = 14) without oral administration of *L. salivarius* V4II-90; and C) GBS-positive women (n = 25) in the probiotic group (10⁹ cfu of *L. salivarius* V4II-90 daily).

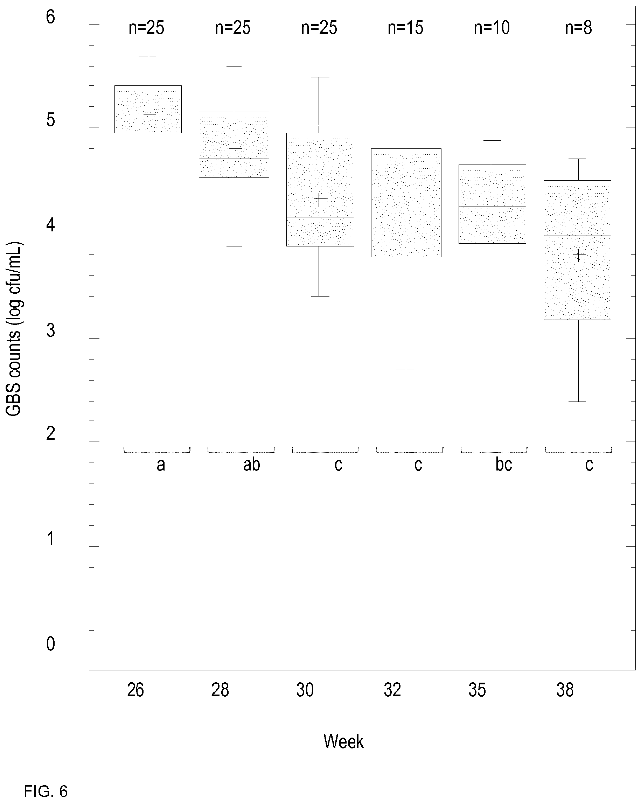

FIG. 6. Means concentration (CFU/mL) of *S. agalactiae* (GBS) in vaginal swabs sampled regularly up to the delivery from GBS-positive women taking10⁹ cfu of *L. salivarius* V4II-90 daily. Statistically significant differences between samples taking at different sampling times are indicated by letters (Bonferroni post-hoc test).

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

It is clear that by using the deposited strain as starting material, the skilled person in the art can routinely, by conventional mutagenesis or re-isolation techniques, obtain further mutants or variants thereof that retain or enhance the herein described relevant features and advantages of the strain of the invention. In one embodiment of the first aspect of the present description, the mutant is obtained by using recombinant DNA technology. In another embodiment of the first aspect of the description, the mutant is obtained by random mutagenesis. Therefore, the description encompasses also a mutant or variant of the deposited *L. salivarius* strain CECT 9145.

A "mutant of a bacterial strain or variant thereof" in the present description encompasses bacteria obtained by mutation, variation or recombination of each of the strains, provided that the resulting bacteria have the ability of being active against *S. agalactiae* infection or able to prevent diseases caused by *S. agalactiae* in animals, including human. That is, the resulting bacteria retain or enhance the activity to treat or prevent early-onset sepsis or late-onset sepsis. In a particular embodiment of the description, the mutant is a genetically modified mutant obtained by classical mutagenesis (i.e. using chemical or physical agents) or by genetic engineering techniques.

The strain of *Lactobacillus salivarius* CECT 9145, or any mutant or variant thereof, as commonly done with microbiological products, can be supplied in form of a bacterial culture of the strain. This culture, as well as the isolated bacteria as defined in the first aspect of the invention, may be used as ingredient of a pharmaceutical composition, as well as an ingredient of any edible product, such as a food supplement.

In a particular embodiment of the first aspect of the invention the isolated strain of *Lactobacillus* is the *Lactobacillus salivarius* deposited at the "Colección Española de Cultivos Tipo (CECT)" under the accession number CECT 9145.

The isolated strain, the bacterial culture and the pharmaceutical composition of the present invention are for use in any mammal, including ruminants and humans.

In one embodiment of the fourth aspect of the invention, optionally in combination with any of the embodiments provided above or below, the infection disease is caused by a *Streptococcus* strain. In another embodiment of the fourth aspect of the invention, optionally in combination with any of the embodiments provided above or below, the infection disease is caused by a *S. agalactiae* strain.

In the present invention the term GBS and *S. agalactiae* are used interchangeably.

In another embodiment of the fourth aspect of the invention, optionally in combination with any of the embodiments provided above or below, the infection disease is sepsis. In another embodiment of the fourth aspect of the invention, optionally in combination with any of the embodiments provided above or below, the infection disease is neonatal sepsis. In another embodiment of the fourth aspect of the invention, optionally in combination with any of the embodiments provided above or below, the infection disease is early-onset sepsis.

In another embodiment of the fourth aspect of the invention, optionally in combination with any of the embodiments provided above or below, the infection disease is a gynecological infection.

Gynecological infections are common among women of all ages. Among the gynecological infections wherein the strain of the first aspect of the invention or the bacterial culture of the second aspect of the invention or the pharmaceutical composition of the sixth aspect of the present invention can be used to treat or prevent them, the following are included: vulvovaginitis, cervical dysplasia, endometritis, endometriosis and intra-amniotic infection.

"Vaginitis" refers to an inflammation of the vagina usually due to infection which can result in discharge, itching and pain, and is often associated with an irritation or infection of the vulva. In the latter case the disorder is termed "vulvovaginitis".

Several types of vaginitis are known to commonly affect women, among them bacterial vaginitis (that can be caused by *S. agalactiae)* and nonspecific vaginitis.

Nonspecific vaginitis is a disease of the vagina which is caused by an imbalance of naturally occurring bacterial flora. A change in normal bacterial flora including the reduction of Lactobacilli, which may be due to the use of antibiotics, hormonal changes or pH imbalance, allows harmful bacteria to gain a foothold and multiply. This type of vaginitis is extremely common, particularly among women of child-bearing age.

Endometritis is the inflammation or irritation of the lining of the uterus (the endometrium). Endometritis is caused by an infection of the uterus and it is more likely to occur after a miscarriage or childbirth. It is also common after prolonged labor or a cesarean section.

Intra-amniotic infection (IAI), also known as chorioamnionitis, is an inflammation of the fetal membranes (amnion and chorion) due to a bacterial infection. It typically results from bacteria ascending into the uterus from the vagina and is often associated with prolonged labor.

Cervical dysplasia refers to abnormal changes in the cells on the surface of the cervix (the lower part of the uterus), considered to be precancerous. Cervical dysplasia can be caused by some carcinogens produced by certain bacteria. Mixed abnormal bacterial flora also might influence the onset and growth of uterine endometrial cancer.

In another embodiment of the fourth aspect of the invention, optionally in combination with any of the embodiments provided above or below, the infection disease is mastitis. In a particular embodiment the mastitis is mastitis of a ruminant; particularly is mastitis of cows. In another particular embodiment, the mastitis is mastitis of a woman.

Mastitis is the inflammation of the breast tissue and causes breast pain, swelling, warmth and redness. Mastitis most commonly affects women who are breast-feeding (lactation mastitis) but can be occur in women who are not breast-feeding. In breast feeding women, it can occur early postpartum or at any point during breast-feeding.

In another embodiment of the fourth aspect of the invention, optionally in combination with any of the embodiments provided above or below, the infection disease is selected from urinary tract infection, bacteremia (presence of bacteria in blood), sepsis (caused by bacteremia), meningitis (inflammation of the tissue that surrounds the brain and spinal cord, called the meninges), endocarditis (also called infective endocarditis, which is an inflammation of the inner lining of the heart), skin and soft tissue infection, or osteomyelitis (infection and inflammation of the bone, can be acute and may evolve into a chronic condition) caused by *S. agalactiae.*

In the present invention the urinary tract infection include infection of kidneys, ureters, bladder, and urethra caused by *S. agalactiae.*

The fourth aspect of the present invention can also be formulated as the use of the isolated strain of the first aspect of the invention or of the bacterial culture as defined in the second aspect of the invention, for the manufacture of a medicament for the prevention and/or treatment of an infection disease.

The fourth aspect of the present invention can also be formulated as a method for the prevention and/or treatment of an infection disease wherein the method comprises the step of administering a therapeutically effective amount of the isolated strain of the first aspect of the invention or of the bacterial culture as defined in the second aspect of the invention, to a subject in need thereof.

In another embodiment of the fourth aspect of the invention, the disease is sepsis, in particular neonatal sepsis, and more particularly early-onset sepsis.

Alternatively, in another embodiment of the fourth aspect of the invention, the disease is a gynecological infection; in a particular embodiment the disease is vulvovaginitis, cervical dysplasia, endometritis, endometriosis or intra-amniotic infection.

Alternatively, in another embodiment of the fourth aspect of the invention, the disease is mastitis. The mastitis can be human, ruminant or cattle mastitis.

In the present invention the terms "subject", "patient" and "individual" are used interchangeably.

In the present invention, the infection caused by *S. agalactiae* occurs in a subject of any age, gender or race.

In the present invention the subject is a mammal, such as a mouse, rat, guinea pig, rabbit, dog, cat, ruminant, horse, primate or human. In a particular embodiment it is a ruminant; in amore particular embodiment it is a cattle, goat or sheep. In another particular embodiment it is a human. In a more particular embodiment, the human is a woman, more preferably a pregnant woman. In another particular embodiment, the subject is a newborn.

In the present invention the term "infection caused by *Streptococcus agalactiae"* comprises asymptomatic infection, urinary tract infections, vulvovaginitis, cervical dysplasia, endometritis, endometriosis, intra-amniotic infection, neonatal infection, and mastitis. In a particular embodiment the infection is early onset sepsis. In another particular embodiment the infection is late onset sepsis. In a particular embodiment, the EOS or LOS neonatal infection comprises respiratory disease, sepsis without focus and/or meningitis, among others.

Infertility and subfertility (the condition of being less than normally fertile though still capable of effecting fertilization) can also be caused by an infection of *Streptococcus agalactiae* in males and females. Therefore, the strain of the first aspect of the invention, or the bacterial culture of the second aspect of the invention, or the pharmaceutical composition of the sixth aspect of the present invention, can be used to treat or prevent infertility and subfertility.

As an adjuvant, the strain of the first aspect of the invention can stimulate the immune system by increasing its response to a vaccine comprising vaccine agents from other infectious diseases.

In this specification, the term "vector" means that the microorganism of the invention may comprise other vaccine antigens from other infectious diseases and act as a delivery system of them.

The strain of the first aspect of the invention, or the bacterial culture of the second aspect of the invention, or the pharmaceutical composition of the sixth aspect of the present invention, can be used either in a subject colonized and/or infected by *S. agalactiae* (for the prevention or treatment of the disease), the subject being an animal including, but not limited to, a human. Thus, the "pharmaceutical composition" can also be a veterinary composition when given to a subject other than a human. In a preferred embodiment the pharmaceutical composition is a veterinary composition for ruminants, especially for cattle and cows.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "excipient" refers to a substance which aids in the absorption of the pharmaceutical composition or medicinal product of the invention, stabilizes said pharmaceutical composition or aids in the preparation thereof in the sense of giving it consistency or providing flavors that make it more palatable or to have a pleasant taste. So the excipients could have the function of keeping the ingredients together, such as starches, sugars or celluloses, for example; the function of sweetening; the function of acting as a dye; the function of protecting the medicinal product such as to isolate it from the air and/or moisture, for example; the function as a filler for a pill, capsule or any other presentation form, without excluding excipients of another type not mentioned in this paragraph.

Another embodiment of the sixth aspect of the invention relates to the pharmaceutical composition that further comprises a preservative

A "preservative" is understood as a substance that maintains the properties of the medicinal product by inhibiting germ contamination; it can be an ionic or non-ionic preservative. The preservative used will not be toxic, will be chemically stable and will be compatible with the strain of the present invention. The preservatives known in the state of the art can be used as preservatives, for example, preservative can refer to benzoic acid, sodium benzoate, ascorbic acid, potassium sorbate, methylparaben, ethylparaben or butylparaben. "Germs" are understood as any cell that can grow and multiply in the composition of the invention that is not the strain of the invention, for example other bacteria, fungi and yeasts.

Another embodiment of the sixth aspect of the invention relates to the pharmaceutical composition that further comprises an antioxidant.

The term "antioxidant" refers to that substance which is capable of delaying or preventing oxidation. Antioxidant agents known in the state of the art can be used as antioxidant agents, for example tocopherol, ascorbic acid, sodium ascorbate, tartaric acid, butylhydroxyanisole, citric acid, vitamin A or vitamin E.

The term "therapeutically effective amount" or "effective amount" as used herein, means an amount of an active agent (ingredient; i.e., a bacterial strain, in the present invention is the strain *L. salivarius* CECT 9145 high enough to deliver the desired benefit, either the treatment or prevention of the illness, but low enough to avoid serious side effects within the scope of medical judgment. The particular dose of compound administered according to this invention will of course be determined by the particular circumstances surrounding the case, including the compound administered, the route of administration, the particular condition being treated, and the similar considerations.

In an aspect of the present invention the therapeutically effective amount is comprised from 10⁸cfu/dose to 10¹⁰cfu/dose. In yet another aspect, it is administered in a dosage regime of 10⁹cfu/dose per day once during several weeks.

In a particular embodiment of the pharmaceutical compositions of the sixth aspect of the invention and of the edible product of the seventh aspect of the invention, the effective amount of the strain is comprised from 10⁸cfu/dose (of composition or edible product) to 10¹⁰cfu/dose of composition or edible product. For the purposes of the invention, any ranges given include both the lower and the upper end-points of the range. Thus, effective amounts in particular embodiments are any multiples from 1 to 9 (1, 2, 3, 4, 5, 6, 7, 8, and 9) of 10⁸cfu/dose, of 10⁹cfu/dose and of 10¹⁰cfu/dose. The amount indicated as cfu/dose relates to the colony forming units of the strain of the invention per dose of pharmaceutical composition or edible product (in particular, food supplement).

In another particular embodiment, the "effective amount" of the strain of the invention is comprised from 10⁸cfu/dose to 10¹⁰cfu/dose, and it is administered or recommended in a dosage regimen from 1 to 3 doses containing the "effective amount" per day. In an embodiment, the strain of the invention is administered in a dosage regime of 10⁹cfu once per day. The effective amount of the strain of the invention can be administered during several weeks to the subject, in a particular embodiment, in a dosage regime of 10⁹cfu/dose per day once during several weeks.

In the case the subject is a pregnant woman colonized or infected by *S. agalactiae,* the dosage regime of the strain of the invention can be administered at any moment of the pregnancy until the *S. agalactiae* is eradicated. The strain of the invention can be administered until delivery of the child. In a particular embodiment, said pregnant woman receives the dosage of the strain of the invention from the 20th week of pregnancy, therefore the first dosage can be administered at 20, 21, 22, 23, 24, 25, 26, 27, 28, 29, 30, 31, 32, 33, 34, 35, 36, 37, 38, 39, 40, 41 or 42 weeks of pregnancy, in a particular embodiment, the pregnant woman receives at least one month of treatment with the strain of the invention.

Said pregnant woman can be colonized or infected by *S. agalactiae* in her gastrointestinal tract or the gynecological tract. The eradication of the *S. agalactiae* in said woman can be measured by routine tests known by the expert in the field in her gastrointestinal tract and/or her gynecological tract.

The pharmaceutical composition may be in the form of a solution, suspension, lyophilized compositions or other dried oral compositions (to be reconstituted with a liquid carrier or directly consumed), tablets, pills, capsules, etc. In a particular embodiment, the pharmaceutical composition is for oral, vaginal, intramammary, rectal, anal, intravenous, intraarterial, parenteral, intracranial, topical, respiratory or nasal administration.

In a more particular embodiment, the pharmaceutical composition can be formulated as a topical composition. Said topical pharmaceutical compositions can be adapted to apply to the skin and mucosa in the form of: a non-ionic vesicular dispersion, emulsion, cream, lotion, gel, aerosol, cream-gel, gel-cream, suspension, dispersion, powder, solid stick, wipe, poultice, foam, spray, oil, ointment, fluid, soap, sanitary towel, ovule, pessary tampon, vaginal suppository or any other form that is known in the art of cosmetics and pharmacy. In a particular embodiment the composition of the invention is formulated as a gel and administered vaginally or cervically by means of a cannula.

In a more particular embodiment, the composition of the invention is for genital administration, particularly to the vulva, vagina or cervix.

In a more particular embodiment the pharmaceutical composition is an intramammary composition to be applied directly to the breast area with inflammation due to mastitis. The skilled in the art will select appropriate carriers and/ or excipients in order to formulate the pharmaceutical composition as desired. Other embodiments include compositions for oral, vaginal, intramammary, rectal, intravenous, intraarterial, parenteral, intracranial, topical, respiratory or nasal route.

Either the composition (of any type) comprising the strain of *L. salivarius* CECT 9145 or a mutant or variant thereof or said strain or the bacterial culture can be in the form of a freeze-dried or lyophilized preparation.

The pharmaceutical composition of the invention may contain adjuvants.

The term "adjuvant" refers to any substance that enhances the response of a given substance. In the present invention, said term refers to any substance that enhances the effects of the pharmaceutical composition of the invention; it can refer to any adjuvant known by the person skilled in the art.

The pharmaceutical composition of the present invention can also comprise a prebiotic, fiber; vitamin, mineral, metal, oligoelement, plant-derived component; fungal-derived component, carotenoid, anti-oxidant, or any combination thereof.

The compositions can comprise a prebiotic and/or a fiber. As used herein, the term "prebiotic" includes substances or compounds that beneficially affect the host mammal by selectively promoting the growth and/or activity of one or more probiotic bacteria in the gastrointestinal tract of the host mammal, thus maintaining normal health or improving health of the host. Typically, prebiotics are carbohydrates, (such as oligosaccharides), but the term "prebiotic" as used herein does not preclude non-carbohydrates. Many forms of "fiber" exhibit some level of prebiotic effect.

Non-limiting examples of prebiotics suitable for use in the composition of the present invention include inulin, psyllium, fructo-oligosaccharides, oligofructose, galacto-oligosaccharides, isomalto-oligosaccharides xylo-oligosaccharides, soy-oligosaccharides, gluco-oligosaccharides, mannan-oligosaccharides, arabinogalactan, arabinxylan, lactosucrose, gluconannan, lactulose, polydextrose, oligodextran, gentioligosaccharide, pectic oligosaccharide, xanthan gum, gum arabic, hemicellulose, resistant starch and its derivatives, and mixtures and/or combinations thereof.

As used herein, the term "fiber" means carbohydrate polymers including those naturally occurring in food as consumed, those having been obtained from food raw material by physical, enzymatic or chemical means, and synthetic carbohydrate polymers, which are resistant to digestion and absorption in the small intestine and have partial fermentation in the large intestine.

Non-limiting examples of fiber and analogous carbohydrate polymers suitable for use in the composition of the present invention include pectins, psyllium, guar gum, xanthan gum, alginates, gum arabic, fructo-oligosaccharides, inulin, agar, betaglucans, chitins, dextrins, lignin, celluloses, non-starch polysaccharides, carrageenan, and mixtures and/or combinations thereof. In one embodiment, the fiber is glucose polymers, preferably those which have branched chains. Other non-limiting examples of suitable fibers include oligosaccharides, such as inulin and its hydrolysis products commonly known as fructo-oligosaccharides, galacto- oligosaccharides, xylo-oligosaccharides, and oligo derivatives of starch.

The fiber can be provided in any suitable form. A non-limiting example is in the form of a plant material which contains the fiber. Non-limiting examples of suitable plant materials include asparagus, artichoke, onion, wheat, chicory, beet pulp, residues of these plant materials, and mixtures thereof.

The compositions of the present invention can optionally comprise one or more vitamins. When certain vitamins, minerals, metals, elements and the like are included as additional components in capsule, tablet and powder forms. The total amount of vitamin(s), by weight, if provided in a premix on or in a suitable carrier, can comprise from about 1% to about 50%, alternatively from about 1% to about 40%, and alternatively from about 2% to about 30%, by weight of the composition. Therefore, when vitamins, minerals, metals and elements are exemplified herein as a % by weight of a composition, such weight % includes any carrier present. With respect to the weight percent of a given vitamin as a percent of a premix or vitamin-carrier mix, such percentages can vary greatly depending on the vitamin and the amount of vitamin desired, as would be understood by one of skill in the art. Generally, however, for vitamins in or on a carrier, the vitamin can comprise, as a weight percent of vitamin to carrier, from about 0.0001% to about 50%, alternatively from about 0.001% to about 45%, alternatively from about 0.001 % to about 40%, by weight of the vitamin-carrier composition.

The compositions of the present invention can comprise Vitamin D. Non-limiting examples of vitamin D suitable for use in the present invention include vitamin D3 (cholecalciferol), vitamin D2 (ergocalciferol) and combinations thereof. Additional non-limiting examples include metabolites of vitamin D including calcidiol, calcitriol and combinations thereof. The vitamin D can be derived from natural or synthetic sources, including from an extract of *Solanum glaucophylum* (malacoxylon), *Trisetum flavescens* (goldhafer) or *Cestrum diurnum.* Both the pure vitamin D and/or glycosides of the vitamin D can be used. The composition of the present invention can comprise from about 50 IU to about 500,000 IU, alternatively from about 500 IU to about 500,000 IU, alternatively from about 1,000 IU to about 500,000 IU of cholecalciferol, per daily dose, alternatively from about 2,000 IU to about 100,000 IU, alternatively from about 10,000 IU to about 50,000 IU, and alternatively from about 20,000 IU to about 40,000 IU, per daily dose, of cholecalciferol.

The pharmaceutical composition can also comprise Vitamin D2 (ergocalciferol). The composition can comprise from about 50 IU to about 500,000 IU, alternatively from about 500 IU to about 500,000 IU, alternatively from about 1,000 IU to about 500,000 IU, and, alternatively from about 5,000 IU to about 500,000 IU of Vitamin D2, per daily dose of the composition. When present, the compositions can comprise from about 1.25µg to about 12.5mg, alternatively from about 125µg to about 12.5mg, alternatively from about 25µg to about 12.5mg, and alternatively from about 125µg to about 12.5mg of vitamin D3 and/or D2, per daily dose of the composition.

The pharmaceutical composition of the present invention can also comprise Vitamin C. The preferred form of Vitamin C for use in the composition is as ascorbic acid or the equivalent of a salt of ascorbic acid or the equivalent of a derivative of ascorbic acid. In a particular embodiment the form of Vitamin C is as calcium ascorbate. The vitamin C may either be in an immediate release form or a sustained release form. When present, the compositions can comprise from about 20mg to about 2000mg, alternatively from about 80mg to about 1500mg, and alternatively from about 100mg to about 1000mg of Vitamin C, per daily dose of the composition.

The pharmaceutical composition of the present invention can also comprise Vitamin A and/or carotene. Vitamin A and carotene can be obtained from either animal or vegetable sources. The animal form is divided between retinol and dehydroretinol whereas the vegetable carotene can be split into four very potent groups - alpha-carotene, beta-carotene, gamma-carotene and crypto- carotene. Non-limiting examples of the Vitamin A useful in the present invention include vitamin A, retinol, retinyl palmitate, retinyl acetate, retinyl proprionate, beta-carotene, alpha carotene, beta-cryptoxanthin, and mixtures thereof. When Vitamin A or one of the forms of Vitamin A is present, the compositions comprise from about 100 IU to about 10,000 IU, alternatively from about 300 IU to about 5,000 IU, alternatively from about 400 IU to about 2,000 IU, and alternatively from about 500 IU to about 1,000 IU of Vitamin A and/or form thereof, per day of the composition. The amount of Vitamin A species may be expressed as IU or as RAE (Retinol Activity Equivalent), which is equal to an equivalent amount of retinol in micrograms. For example, 10,000 IU Vitamin A is equivalent to 3000 RAE or 3000 µg retinol. When Vitamin A (retinol) is present, the compositions can comprise from about 30µg to about 4545µg, alternatively from about 90µg to about 1500|jg, alternatively from about 120µg to about 600µg, and alternatively from about 150µg to about 300µg of Vitamin A (retinol), per daily dose of the composition.

The pharmaceutical composition of the present invention can comprise one or more B Vitamins. Non-limiting examples of Vitamin B useful in the present invention include vitamin B1 (thiamin), Vitamin B2 (Riboflavin), Vitamin B3 (niacin), Vitamin B5 (pantothenic acid), Vitamin B6 (pyridoxine, pyridoxal, or pyridoxamine), Vitamin B7 (Biotin), Vitamin B9 (Folic acid), Vitamin B 12 (cyanocobalamin), and mixtures thereof.

When Vitamin B1 is present, the compositions can comprise from about 200µg to about 50mg, alternatively from about 400µg to about 20mg, and alternatively from about 500µg to about 10mg of Vitamin B1, per daily dose of the composition.

When Vitamin B2 is present, the compositions can comprise from about 100µg to about 200mg, alternatively from about 200µg to about 100mg, and alternatively from about 500µg to about 50mg of Vitamin B2, per daily dose of the composition.

When Vitamin B3 is present, the compositions can comprise from about 1mg to about 500mg, alternatively from about 2mg to about 250mg, and alternatively from about 5mg to about 100mg of Vitamin B3, per daily dose of the composition.

When Vitamin B5 is present, the compositions can comprise from about 500µg to about 1000mg, alternatively from about 1000µg to about 500mg, and alternatively from about 2000µg to about 100mg of Vitamin B5, per daily dose of the composition.

When Vitamin B6 is present, the compositions can comprise from about 200µg to about 500mg, alternatively from about 500µg to about 250mg, and alternatively from about 1000µg to about 100mg of Vitamin B6, per daily dose of the composition.

When Vitamin B7 is present, the compositions can comprise from about 1µg to about 200µg, alternatively from about 2µg to about 100µg, and alternatively from about 5µg to about 50µg of Vitamin B7, per daily dose of the composition.

When Vitamin B9 is present, the compositions can comprise from about 50µg to about 2000µg, alternatively from about 100µg to about 1000µg, and alternatively from about 200µg to about 500µg of Vitamin B9, per daily dose of the composition.

When Vitamin B12 is present, the compositions can comprise from about 0.5µg to about 3000µg, alternatively from about 1 µg to about 1500µg, and alternatively from about 2µg to about 750µg of Vitamin B 12, per daily dose of the composition.

The pharmaceutical compositions of the present invention can include minerals, metals and elements. Non-limiting examples of minerals, metals, and elements useful in the compositions of the present invention include: iron, iodine, zinc, copper and selenium. When present, the minerals, metals and/or elements are comprised from about 1% to about 50% by weight of the composition and alternatively from about 2% to about 30%, by weight of the composition.

As used herein plant-derived components can include herbs including those used in traditional native American, Chinese and Japanese medicine, herbal extracts, and isolated active components of plants from the flower, leaves, stems, roots, and seeds of plants.

The pharmaceutical composition of the present invention can also comprise fungal-derived components that are known to have beneficial effects with respect to enhancing immune response to respiratory conditions. Non-limiting examples of such fungal-derived components include the ones obtained from: Maitake and Shiitake mushrooms (*Grifola frondosa* and *Lentinus edodes* respectively) and Reishi mushroom (*Ganoderma lucidum*); yeast (*Saccharomyces cerevisiae, Saccharomyces boulardii*) and cell wall extracts of yeast cells and molds, The fungal-derived components can include whole, ground, crude-sized, and superfine particle extracts, micellary extracts, and mixtures thereof, of the fungus. In a particular embodiment the fungal-derived component can comprise an extract of edible mushroom.

The pharmaceutical composition of the present invention can comprise at least one polyphenol. Non- limiting examples of sources of polyphenols useful in the present invention include tea extract, rosemary extract, rosmarinic acid, coffee extract, caffeic acid, turmeric extract, blueberry extract, grapeseed extract, and mixtures thereof. Polyphenols have antioxidant activity and anti-inflammatory effects. When present, the compositions can comprise from about 0.01% to about 90%, alternatively from about 0.1% to about 35%, alternatively from about 1% to about 15%, alternatively from about 1% to about 10%, and alternatively from about 3% to about 10% of a polyphenol, by weight of the composition.

The pharmaceutical composition of the present invention can comprise a carotenoid. When a carotenoid is present, the carotenoid is selected from the group consisting of lutein, astaxanthin, zeaxanthin, bixin, lycopene, and mixtures thereof.

When an antioxidant is present, non-limiting examples of such antioxidants include tocopherols (Vitamin E), Vitamin C, Vitamin A, CoQ10, plant-derived materials carotenoids, selenium, or any combination thereof.

The strain of the present invention (*Lactobacillus salivarius* CECT 9145) (or any mutant or variant thereof of the description), has probiotic properties. It is then a probiotic strain with the capacity of treating and/or preventing an infection caused by *S. agalactiae.* As probiotic, this strain (or any mutant or variant thereof) is able to resist the environment of the gastrointestinal tract, adheres well to the gut epithelium (as demonstrated with the adhesion to Caco-2 cells and HT-29 cells), does not produce toxic compounds, it has anti-*S*. *agalactiae* activity and is no toxic to animals.

The term "probiotic" is to be understood in the sense of the present invention as live microorganisms which, when administered in adequate amounts, confer a health benefit on the host. The known benefits of enteral administration of probiotic microorganisms include enhanced host defense to disease improving the properties of the indigenous microbiota and increasing colonization resistance to the harmful microbiota. Probiotics have been suggested to play an important role in the formation or establishment of a well-balanced, intestinal microbiota in either infants or adults receiving high doses of antibiotics. Lactic acid bacteria and Bifidobacterium, especially specific strains of the Genus *Lactobacillus* have been recommended for their use as probiotics.

The strain of the *L. salivarius* CECT 9145, any mutant or variant thereof, as well as any culture which comprises it, can be an ingredient of any edible product or composition, therefore, it can be together with appropriate amounts of other edible ingredients. In this edible product or composition, the strain of *L. salivarius* CECT 9145 will be in an effective amount to promote the desired effect. As above indicated, the effective amount is in particular comprised from (and including) 10⁸cfu/dose of edible product to 10¹⁰cfu/dose of edible product, preferably 10⁹cfu/dose. In a particular embodiment, the edible product is selected from the group consisting of a milk product, a yogurt, a curd, a cheese, a fermented milk, a powdered milk, a milk based fermented product, a meat based fermented product, an ice-cream, a cereal based fermented product, a beverage, a snack, a flour, a chewing-gum, a sweet, a sweet food, a pet food, veterinary food, a dietary or food supplement, a functional food, nutraceutical, enriched food, a clinical nutrition formula, a nutritional complement, a formula for pregnant woman, a formula for the elderly and an infant formula (including newborn and preterm formula).

In a particular embodiment is a ruminant food, more particularly, cattle food.

In a particular embodiment, the edible product is a food supplement comprising an effective amount of the strain as defined above (*L*. *salivarius* CECT 9145) as active ingredient together with appropriate amounts of other edible ingredients. In a particular embodiment, said effective amount of the strain is comprised from 10⁸cfu/dose of composition to 10¹⁰cfu/dose of composition. In another particular embodiment, the food supplement comprises an effective amount of the strain consisting in *L. salivarius* CECT 9145. In another particular embodiment, the food supplement comprises an effective amount from 10⁸cfu/dose of composition to 10¹⁰cfu/dose of composition of the strain consisting in *L. salivarius* CECT 9145.

The term "food supplement" or "dietary supplement" are used herein as synonymous and relate to edible compositions intended to provide nutrients that may otherwise not be consumed in sufficient quantities or that are missing (in for example, infants, pregnant women, elderly people, etc.). These supplements include, among others, vitamins, minerals, fatty acids, fiber and amino acids.

The term "nutraceutical" refers to a food (or part of a food) that provides medical or health benefits, including the prevention and/or treatment of a disease.

The pharmaceutical composition of the present invention can also comprise at another probiotic strain of bacteria or yeast, or any combination thereof, such as any member of the genus Bifidobacterium, Lactobacillus, Streptococcus, Lactococcus, Bacillus, Torulaspora and yeasts, with known probiotic properties.

Among the Lactobacillus, non-limiting examples can be *Lactobacillus acidophilus, L. bulgarius, L. gasseri, L. plantarum, L casei, L. johnsonii, L. gallinarum, L. amylovorus, L. brevis, L. rhamnosus, L. paracasei, L. crispatus, L. helveticus, L. reuteri, L. GG, L derbrueckii, L. fermentum, L. lactis, L. vaginalis, L. gastricus, L. antri, L. kalixensis, L. kefiranofaciens* and *L. ultunensis.*

The pharmaceutical composition can comprise another strain of *L. salivarius.*

Among the Bifidobacterium genus, non-limiting examples can be *Bifidobacterium. bifidum, B. longum, B. adolescentis, B. infantis, B. breve, B. catenulatum, B. animalis, B. angulatum, B. rumiantum, B. gallicum, B. magnum, B. chocrinum, B. cuniculi, B. pseudolongum, B. suis, B. pseudocatenulatum, B. angulatum, B. merycicum, B. gallinarum, B. pullorum, B. boum, B. thermacidophillum, B. thermophillum, B. minimum, B. indicum, B. coryneforme* and *B*. *asteroides.*

As explained above, other microorganisms that can be comprised in the pharmaceutical composition are those from the Streptococcus, Lactococcus, Bacillus or Torulaspora genus or yeasts, provided that they display probiotic properties, such as *Streptococcus thermophilus, Lactococcus lactis, Bacillus subtilis, Saccharomyces cerevisiae, Saccharomyces boulardii, Torulaspora delbrueckii, T. hansenii, T. lactis, T. marxianus* and *T. lodderae.*

Additionally, the strain as defined above, that is, the *L. salivarius* CECT 9145 or a mutant or variant thereof, as well as cultures comprising the same, can also be used in a medical device once formulated as ingredient in a product considered by the authorities as a medical device or medical device composition. Thus, the strain as defined above can be applied directly or as an ingredient of a medical device. The medical device comprises an effective amount of the strain of the invention together with any acceptable excipient and/or carrier and/or any other components defining the medical device (i.e.: any support, or woven or non-woven fabric). In a particular embodiment, the medical device comprises an effective amount of the strain consisting in *L. salivarius* CECT 9145.

The term "medical device" means as defined by the European Directive 2007/47/EC any product, instrument, device, apparatus, computer program, material or article used in the medico sanitary field and being regulated by the European Directive 90/385/CEE of active implantable medical device, 98/79/CE of medical devices for *in vitro* diagnostic, and 93/42/CEE for general medical devices. The medical devices are included in the sanitary technologies. They are used in human beings for the diagnostic, prevention, control, treatment or alleviation of a disease, for the compensation of a deficiency; for investigating, substituting or modification of anatomy or a physiologic process; and for the regulation of contraception. Medical devices of the present invention relate to compositions or formulations ("medical device compositions"), or to products or devices comprising said compositions or formulations, comprising an effective amount of the strain as defined above (*L*. *salivarius* CECT 9145) as active ingredient. The term "medical device composition" is used herein to identify the compositions according to the invention, which compositions, as such (by their selves) are catalogued by the competent sanitary authorities as medical devices.

The medical device of the present invention allows the application or administration of the strain of the invention to a desired tissue surface, for example, in order to pharmaceutically and/or via the medical device itself treat or take care of the surface to which it is applied. In some embodiments, the composition comprising the strain of the invention is *per se* catalogued by the authorities as a medical device. In other particular embodiments of the medical devices, they correspond to sanitary articles that include woven or non-woven fabrics, in which the strain of the invention is homogeneously distributed along the surface of the fabric. This distribution can be performed, optionally, with the aid of a solvent. The solvent may also optionally be dried after layer or coating of the composition comprising the effective amount of the strain, is totally or partially distributed onto the surface of the fabric, thus obtaining fabrics with the properties conferred by the strain according to the invention.

An ninth aspect of the present invention relates to a method to obtain a mutant of the strain of *L. salivarius* CECT 9145, comprising using the deposited strain as starting material and applying mutagenesis, wherein the obtained mutant retains or enhances the activity of the parent deposited strain to prevent or treat diseases caused by *S. agalactiae.* The methods to obtain a mutant are the ones known by the expert in the field.

A tenth aspect refers to a kit that comprises an effective amount of the strain of the first aspect of the invention, the bacterial culture of the second aspect of the invention or the pharmaceutical composition of the sixth aspect of the invention.

In a particular embodiment the kit may comprise a weekly, monthly, or other periodic dose of the strain of the first aspect of the invention. As an illustrative example, a kit comprising a weekly dose may comprise seven discrete compositions comprising the probiotic (seven daily doses). As another example, a kit comprising a monthly dose may comprise thirty discrete compositions comprising the strain of the first aspect of the invention.

In case the strain of the first aspect of the invention is lyophilized, the kit of the invention can contain an edible resuspension agent, such as water.

In a particular embodiment the kit is configured to facilitate dosing compliance. For example, the kits may be particularly advantageous for the purpose of ensuring that the subject is receiving administration of the effective amount of the strain of the invention on the appropriately prescribed schedule. Blister cards or other containing devices appropriately configured may be particularly suitable for clearly illustrating sequence or timing of administration of the various components. The kit may be obtained as one card, or cases of four, six, seven (e.g., a weekly supply), or eight cards co-packaged together. Additionally, monthly or other types of kits may be obtained.

The kit that comprises the bacterial culture of the second aspect of the invention can comprise any means that allows the correct culture of the strain of the invention, such as culture medium, supplements or antibiotics.

The present invention also refers to the use of the kit of the tenth aspect of the invention for the treatment and/or prevention of a disease, preferably caused by a *Streptococcus* strain, more preferably by *S. agalactiae,* wherein in a particular embodiment any of the diseases mentioned above, in a more particular embodiment is sepsis, more preferably neonatal sepsis, and more preferably is early-onset sepsis. In another particular embodiment the disease caused by *S. agalactiae* is gynecological infection or mastitis (wherein in an embodiment is mastitis in ruminants, such as cattle and cow and in another embodiment is human mastitis). The present invention also refers to the use of the isolated strain, the bacterial culture, the pharmaceutical composition and the edible product of the present invention in the prevention and/or treatment of an infection disease, preferably caused by a *Streptococcus* strain, in a particular embodiment the infection disease is caused by *S. agalactiae,* in a more particular embodiment in any of the infection diseases described herein.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples:

### Materials and methods:

### 1. Assessment of the vaginal microbiota of GBS-positive and GBS-negative fertile women (non-pregnant and pregnant women)

### 1.1. Participating women

A total of 54 fertile women (30 non-pregnant women and 24 pregnant women), aged 25-35 participated in this study. All volunteers gave written informed consent to the protocol, which had been approved (protocol 10/017-E) by the Ethical Committee of Clinical Research of Hospital Clínico San Carlos Madrid (Spain).In relation to non-pregnant women, 4 vaginal exudate samples were collected within a menstrual cycle (days 0, 7, 14 and 21). Pregnant women provided a single sample in week 35-37 of pregnancy. All women claimed to be completely healthy.

### 1.2. Microbial isolation, enumeration and identification

Samples were diluted in peptone water and spread onto Columbia Nalidixic Acid (CNA), Mac Conkey (MCK), *Sabouraud* Dextrose Chloramphenicol (SDC), Gardnerella (GAR) and Mycoplasma agar plates (BioMerieux, Marcy l'Etoile, France) for selective isolation and quantification of the main agents involved in vaginal infections. They were also spread onto agar plates of MRS (Oxoid, Basingstoke, UK) supplemented with either L-cysteine (0.5 g/L) (MRS-C) or horse blood (5%) (MRS-B) for isolation of lactobacilli. The plates were incubated for 48 h at 37°C in aerobic conditions, except for the MRS-C and MRS-B ones, which were incubated anaerobically (85% nitrogen, 10% hydrogen, 5% carbon dioxide) in an anaerobic workstation (DW Scientific, Shipley, UK). Parallel, all the simples were enriched in Todd Hewitt broth (Oxoid, Basignstoke, UK) to facilitate the isolation of Streptococcus agalactiae.

Initially, identification of the bacterial strains was performed by sequencing the 470 base pair (bp) fragment of the 16S ribosomal deoxyribonucleic acid (rDNA) gene PCR (Polymerase Chain Reaction) amplified using the primers pbl16 (5'-AGAGTTTGATCCTGGCTCAG-3') (SEQ ID NO: 1) and mbl16 (5'-GGCTGCTGGCACGTAGTTAG-3') (SEQ ID NO: 2) (Kullen *et al*., 2000). The PCR conditions were as follows: 96 °C for 30 s, 48 °C for 30 s and 72 °C for 45 s (40 cycles) and a final extension at 72 °C for 4 min. The amplicons were purified using the Nucleospin® Extract II kit (Macherey-Nagel, Düren, Germany) and sequenced at the Genomics Unit of the Universidad Complutense de Madrid, Spain. The resulting sequences were used to search sequences deposited in the EMBL database using BLAST algorithm and the identity of the isolates was determined on the basis of the highest scores (>99%).

Identification of yeasts and confirmation of the initial 16S rDNA identifications was performed by MALDI-TOF (VITEK MS, BioMerieux) in the facilities of Probisearch (Tres Cantos, Spain). Briefly, a portion of a bacterial colony (∼1 µL) was directly spotted onto a MALDI sample plate. Then, it was overlaid with 1 µL of a saturated solution of α-cyano-4-hydroxycinnamic acid in acetonitrile (28%), and allowed to dry at room temperature. For each isolate, a mean spectrum was constructed with at least 50 m/z spectra profiles and used for the identification by comparison with the spectra contained in the Myla database (Biomerieux). Identification was defined as a 99-100% match to the species-specific m/z values in the database.

Identification of *S. agalactiae* isolates was also confirmed by using a latex agglutination test (Streptococcal grouping kit, Oxoid).

A total of 89 different *Lactobacillus* strains were isolated from the vaginal swabs and, subsequently, submitted to Random Amplification of Polymorphic DNA (RAPD) genotyping in order to avoid duplication of isolates. RAPD profiles were obtained using primer OPL5 (5'-ACGCAGGCAC-3') (SEQ ID NO: 3), as described by Ruiz-Barba *et al.* (2005).

*Lactobacillus* strains were routinely cultured in MRS-C medium at 37 °C under aerobic conditions. When required, they were grown in anaerobiosis as described above. *Lactobacillus* strains were stored at 80°C in MRS-C broth containing 15% glycerol (vol/vol).

### 2. Antimicrobial activity against S. agalactiae strains

An overlay method (Magnusson and Schnürer, 2001) was used to determine the ability of the lactobacilli strains to inhibit the growth of 12 different *S. agalactiae* strains, 6 isolated from blood or cerebrospinal fluid in clinical cases of neonatal sepsis (Hospital Universitario Ramon y Cajal, Madrid, Spain) and the remaining 6 from vaginal samples of pregnant women (our own collection). It was performed using MRS agar plates, on which the lactobacilli strains were inoculated as approximately 2-cm-long lines and incubated at 37°C for 48 h. The plates were then overlaid with the indicator *S. agalactiae* strains vehiculated in 10 ml of Brain Heart Infusion (BHI, Oxoid) broth supplemented with soft agar (0.7%), at a concentration of ∼10⁴ colony-forming units (cfu)/ml. The overlaid plates were incubated at 37°C for 48 h and, then, examined for clear zones of inhibition (> 2 mm) around the lactobacilli streaks. All experiments assaying inhibitory activity were performed in triplicate.

### 3. Production of bacteriocins

The *Lactobacillus* strains were grown in MRS broth at 37°C until early stationary phase (A₆₂₀ ∼1.0). The culture was centrifuged at 12000×*g* for 10 min at 4°C, and the supernatant was neutralized to 6.2 with 1 M NaOH, heated at 100°C for 5 min and filter-sterilized through 0.22 µm-pore-size filters (Millipore, Bedford, USA). The bacteriocinogenic activity of the cell-free supernatants was determined by an agar well diffusion assay. Aliquots (100 µl) of the supernatants were placed in wells (7-mm diameter) cut in cooled BHI agar plates previously seeded (10⁵ cfu/ml) with the *S. agalactiae* indicator strains. The plates were kept at 4°C for 2 h and, then, incubated under optimal conditions for growth of the indicator.

### 4. Production of hydrogen peroxide

Hydrogen peroxide production by the *Lactobacillus* strains was initially tested following the procedure described by Song *et al.* (1999). MRS agar plates supplemented with 0.25 mg/ml of tetramethylbenzidine (TMB; Sigma, St. Louis, USA) and 0.01 mg/ml of horseradish peroxidase (HRP, Sigma) were inoculated with the strain and anaerobically incubated for 2 days at 37°C. HRP is known to oxidize TMB in the presence of hydrogen peroxide to form a blue pigment in the H₂O₂-producing colony. Parallel, the hydrogen peroxide was also measured by a modification of the quantitative method of Yap and Gilliland (2000). The strain was anaerobically grown in 10 ml of MRS broth for 24 h at 37°C. The cells were harvested by centrifugation at 12000 × *g* for 10 min at 4°C, washed twice with potassium phosphate buffer (50 mM, pH 6) and resuspended in 9 ml of the same buffer supplemented with 5 mM glucose. The cell suspension (0.5 ml) was inoculated into a tube containing 9 ml of the glucose-containing buffer. After an incubation at 37°C for 24 h in aerobiosis, the cells were removed by centrifugation at 12000 ×*g* for 10 min at 4°C and the supernatants were assayed for hydrogen peroxide. Briefly, 5 ml of supernatant were mixed with 100 µL of 1% aqueous o-dianisidine (Sigma), and 1 ml of 0.001% aqueous HRP. The tubes were incubated for 10 min at 37°C and the reaction was stopped by adding 0.2 ml of 4 N HCl. Absorbance reading (A_{400 nm}) was determined and peroxide content was quantified by comparing the values obtained with those of a H₂O₂ standard curve. In the H₂O₂ assays, *Lactobacillus acidophilus* CECT 903^{T} and *Lactobacillus gasseri* CECT 5714 were used as a positive control.

### 5. Production of lactic acid

Lactic acid production by the *Lactobacillus* strains was determined in MRS broth (pH 6.2). One percent inoculum's from an overnight MRS culture was used and incubation proceeded for 24 h at 37°C anaerobically. Cells were removed by centrifugation at 12000 × *g* for 5 min and the concentration of L- and D-lactic acid in the supernatants was quantified using an enzymatic kit (Roche Diagnostics, Mannheim, Germany), following the manufacturer's instructions. The pH values of the supernatants were also measured. All these assays were performed in triplicate and the values were expressed as the mean ± SD. *L. salivarius* CECT 5713 was used as a positive control (high production of L-lactic acid in MRS broth) (Martin *et al*., 2006).

### 6. Co-aggregation between the lactobacilli and the S. agalactiae strains

Coaggregation assays were designed based on previously reported methods (Reid *et al*., 1990). Bacterial suspensions were adjusted to an A₆₀₀ of 0.6. Aliquots of 10 ml of each of the *Lactobacillus* strains were independently mixed with 10 ml of each of the *S. agalactiae* strains and incubated at 37°C for 4 h and 16 h. The suspensions were then observed under a phase-contrast microscope after Gram staining

### 7. Broth co-cultures of the lactobacilli and the S. agalactiae strains

To test the anti-*S*. *agalactiae* activity of the lactobacilli in a broth assay format, tubes containing 20 ml of MRS broth were co-inoculated with 1 mL of a *Lactobacillus* strain culture (7 log₁₀ cfu/mL)and 1 ml of a *S. agalactiae* strains (7 log₁₀ cfu/mL). Subsequently, the cultures were incubated for 6 h at 37°C in aerobic conditions. Immediately after the co-inoculation and after the incubation period, aliquots were collected, serially diluted and plated on MRS-C plates and CHROMagar StrepB agar plates (CHROMagar, Paris, France) for selective enumeration of lactobacilli and streptococci, respectively. Correct taxonomic assignment was confirmed by MALDI-TOF analysis as described previously.

### 8. Adherence assays to intestinal and vaginal epithelial cells

The adherence of lactobacilli to HT-29 and Caco-2 cells was examined as described by Coconnier *et al.* (1992). Routinely, cells were grown in DMEM medium (PAA, Linz, Austria) containing 25 mM glucose, 1 mM sodium pyruvate and supplemented with 10% heat-inactivated (30 min, 56°C) fetal calf serum, 2 mM L-glutamine, 1% non-essential amino acid preparation, 100 U/mL penicillin and 100 mg/mL streptomycin. For the adherence assays, HT-29 and Caco-2 were cultured to confluence in 2 mL of medium devoid of antibiotics. Approximately 10 days postconfluence, 1 mL of the medium was replaced with 1 mL of *Lactobacillus* suspension (10⁸ cfu/mL in DMEM). The inoculated cultures were incubated for 1 h at 37°C in 5% CO₂. Then, the monolayer was washed five times with sterile PBS, fixed with methanol, stained with Gram stain and examined microscopically. The adherent lactobacilli in 20 random microscopic fields were counted for each test. *Lactobacillus rhamnosus* GG and *Lactobacillus casei* imunitass were used as positive and negative controls because of their high and low adhesive potential, respectively (Martin *et al*., 2005).

Adherence to vaginal epithelial cells collected from healthy premenopausal women was performed as described previously (Boris *et al*., 1998).

### 9. Adherence to and/or degradation of mucin

The adhesion of the lactobacili strains to mucin was determined according to the method described by Cohen and Laux (1995) with some modifications. Briefly, 100 µl of a solution (1 mg/ml) of porcine mucin (Sigma) in HEPES-buffered Hanks salt solution (HH) were immobilized in polystyrene microtiter plates (Maxisorp; Nunc, Roskilde, Denmark) after overnight incubation at 4°C. The wells were washed twice with 250 µl of HH. Parallel, bacteria were grown overnight at 37°C in MRS broth and the bacterial pellets from 1 ml fractions were obtained by centrifugation and washed with HH. Then, 10 µl of 10 mM carboxyfluorescein (Sigma) were added to the pellets and the bacterial suspensions were incubated for 20 min at 37°C. Subsequently, the bacterial cells were washed 3 times with HH and, finally, resuspended in 1 ml of HH. Then, a suspension of 50 µl of the fluorescent-labelled bacteria (∼ 5×10⁷ cfu) was added to each well. After incubation for 1 h at 37°C, the plates were washed twice with 250 µl of HH to remove unattached cells, and incubated for 1 h at 60°C in the presence of 50 µl of 1% sodium dodecyl sulphate (SDS)-0.1 M NaOH to release and lyse bound microorganisms. Fluorescence was measured in a fluorescence microplate reader (Tecan Austria GMBH, Salzburg, Austria). Adhesion was assessed as the percentage of the fluorescence retained in the wells after the washing steps when compared to that present in the labeled bacterial aliquots originally added to the wells. *L. reuteri* CR20 was used as a positive control (strong adherence to mucin) (Martin *et al*., 2009). The assays were performed in duplicate.

The potential of the lactobacilli strains to degrade gastric mucine (HGM; Sigma) *in vitro* was evaluated in duplicate following the procedure developed by Zhou *et al.* (2001).

### 10. Survival after transit through an in vitro gastrointestinal model

The survival of the strains was tested in an *in vitro* model of the human stomach and small intestine based on that described by Marteau *et al.* (1997). Aliquots (25 ml) containing approximately 10⁹ cfu/ml of the strain tested was diluted in 5 ml of a sterile electrolyte solution containing 6.2 g/l of NaCl, 2.2 g/l of KCI, 0.22 g/l of CaCl₂, and 1.2 g/l of NaHCO₃ to simulate the *in vivo* dilution by saliva. Then, 5 ml of porcine gastric juice was added and the mixture was incubated at 37°C with agitation. The pH curve in the stomach-resembling compartment was controlled to reproduce the values found in monogastrics after yogurt consumption (Conway *et al.* 1987): pH 5.0 at initiation, pH 4.1 at 20 min, pH 3.0 at 40 min, and pH 2.1 at 60 min. Fractions were successively taken from this compartment at 20, 40, 60, and 80 min, in a manner that simulates the normal gastric emptying (Marteau *et al.* 1997). After adjusting their pH to 6.5 ± 0.2 with 1 M NaHCO₃, they were mixed with 10 ml of a sterile electrolyte solution containing 5 g/l of NaCl, 0.6 g/l of KCl, 0.3 g/l of CaCl₂, 4% of porcine bile, and 7% of pancreatin (Sigma), which simulates the content of the duodenal juice. After 120 min of successive exposure to these conditions, bacterial survival was determined by plating the samples onto MRS agar plates, which were anaerobically incubated at 37°C for 48 h. *L. salivarius* CELA2 was used as a positive control because of its high resistance to gastrointestinal-like conditions (Martin *et al.,* 2009). All these assays were performed in quadruplicate and the values were expressed as the mean ± SD.

### 11. Antibiotic resistance/susceptibility

Minimum inhibitory concentrations (MICs) of the 16 antibiotics included in this study (gentamicin, kanamycin, streptomycin, neomycin, tetracycline, erythromycin, clindamycin, chloramphenicol, ampicillin, penicillin, vancomycin, virginiamycin, linezolid, trimethoprim, ciprofloxacin and rifampin) were determined by a microdilution method using VetMIC plates for lactic acid bacteria (National Veterinary Institute of Sweden, Uppsala, Sweden), as described previously (Langa *et al.* 2012). The plates were incubated at 37°C for 48 h and MIC was defined as the lowest concentration at which no growth was observed. MICs values were compared with the microbiological cut-off parameters established by the European Food Safety Authority (EFSA, 2012) for *Lactobacillus* species.

### 12. Prophage induction

In order to discard the presence of phages compromising strain viability, prophage induction was carried as described previously (Langa *et al.* 2012). Exponential cultures of the lactobacilli strains (∼OD₆₀₀ 0.4) were treated with 0.25, 0.5 (minimal inhibitory concentration; MIC), and 1 µg/ml mitomycin C (final concentration), and incubation was continued for up to 5 h. Aliquots of the supernatants were placed on lawns of presumably susceptible *L. salivarius* strains growing in soft MRS (0.75% agar) supplemented with 1% haemoglobin, 10 mM CaCl₂ and 10 mM MgSO₄, placed on top of plates with the same medium (1.5% agar). After incubation for 24 h, the generation (or not) of lysis plates was recorded.

### 13. Production of biogenic amines

The ability to form biogenic amines (tyramine, histamine, putrescine and cadaverine) was assessed using the decarboxylase broth and the method described by Bover-Cid and Holzapfel (1999). The precursor amino acids (tyrosine, histidine, ornithine and lysine, respectively) were purchased from Sigma. The lactobacilli strains were streaked onto the different decarboxylase medium plates and incubated for 4 days at 37°C under aerobic and anaerobic conditions. A positive result was indicated by a change of the medium color to purple in response to the pH shift caused by the production of the more alkaline biogenic amine from the amino acid initially included in the medium. Parallel, the lactobacilli strains were grown for 24 h in MRS broth supplemented with 10 mM tyrosine (M17T), 13 mM of histidine (M17H) or 20 mM agmatine (M17A) for the detection of tyramine, histamine and putrescine production, respectively. The supernatants were filtered through a 0.2 µm pore diameter membrane, derivatyzed and analyzed by thin layer chromatography (TLC) following the conditions described by Garcia-Moruno *et al* (2005).

In addition, the presence of the tyrosine decarboxylase gene (*tdcA*), histidine decarboxylase gene (*hdcA*) and agmatine deiminase cluster *(AgdDI)* was checked by specific PCR using methods described previously (Le Jeune *et al*., 1995; Lucas and Lonvaud-Funel, 2002; Ladero *et al*., 2012).

### 14. In vivo safety assessment of L. salivarius V4II-90

Globally, *L. salivarius* V4II-90 was the strain that showed the best results as a candidate for future clinical trials. Subsequently, its potential acute and repeated dose (4 weeks) oral toxicity was evaluated; in addition, the potential translocation of this strain to blood and some organs was also investigated.

### 14.1. Acute and repeated dose (4-weeks) oral toxicity studies

Wistar male and female rats (Charles River Inc., Marget, Kent, UK) were acclimated for 7 days prior to study initiation with an evaluation of health status. The rats were individually housed in polycarbonate cages with sawdust bedding and maintained in environmentally controlled rooms (22 ± 2 °C and 50% ± 10% relative humidity) with a 12 h light-dark cycle (light from 08.00 to 20.00 h). Food (A03 rodent diet, Scientific Animal Food and Engineering, Villemoisson-sur-Orge, France) and water were available *ad libitum.* The rats were 56-days old at the initiation of treatment.

Acute (limit test) and repeated dose (4 weeks) studies were conducted in accordance with the European Union guidelines (EC Council Regulation No. 440, 2008a,b). Both studies were undertaken in accordance with the ethics requirements and authorized by the Official Ethical Committee of the Complutense University.

In the acute (limit test) study, 24 rats (12 males, 12 females) were distributed into two groups of 6 males and 6 females each. After an overnight fast each rat received skim milk (500 µl) orally (control group or Group 1), or a single oral dose of 1 × 10¹⁰ colony-forming units (cfu) of *L salivarius* V4II-90 dissolved in 500 µl of skim milk (treated group or Group 2). Doses of the test and control articles were administered by gavage. Animals were checked for clinical signs and mortality twice a day (a.m. and p.m.). At the end of a 14 days observation period, the rats were weighed, euthanized by CO₂ inhalation, exsanguinated, and necropsied.

The repeated dose (4 weeks) (limit test) study was conducted in 48 rats (24 males, 24 females) divided in four groups of 6 males and 6 females each (control group or Group 3; treated group or Group 4; satellite control group or Group 5; and satellite treated group or Group 6). Rats received a daily dose of either skim milk (Groups 3 and 5) or 1 × 10⁹ cfu of *L salivarius* V4II-90 dissolved in 500 µl of skim milk (Groups 4 and 6) orally once a day over 4 weeks. Doses of the test and control articles were administered by gavage. Animals were dosed at approximately the same time each day (approximately 4-6 h into light cycle). Food but not water was withheld from 4 h before until 2 h after control and test article administration. Animals were checked for clinical signs and mortality twice a day (a.m. and p.m.). All rats of the Groups 3 and 4 were deprived of food for 18 h, weighed, euthanized by CO₂ inhalation, exsanguinated, and necropsied on Day 29. All animals of the satellite groups (Groups 5 and 6) were kept a further 14 days without treatment to detect delayed occurrence, or persistence of, or recovery from toxic effects. All rats of the Groups 5 and 6 were deprived of food for 18 h, weighed, euthanized by CO₂ inhalation, exsanguinated, and necropsied on Day 42.

### 14.2. Animal observations

All animals were observed twice daily for general appearance, behaviour, sings of morbidity and mortality (once before treatment and once daily thereafter). Rats were observed for their general condition and the condition of the skin and fur, eyes, nose, oral cavity, abdomen and external genitalia, evaluated for respiration rate and palpated for masses. Behavioural parameters checked were abnormal movements (tremor, convulsion and muscular contractions), reactions to handling and behaviour in open field (excitability, responsiveness to touch and to sharp noise), changes in ordinary behaviour (changes in grooming, head shaking, gyration), abnormal behaviour (autophagia, backward motion) and aggression. Body weight, body weight gain and food and water consumption were measured daily and at the end of the observation periods the rats were examined by necropsy, and the weights of the organs recorded.

### 14.3. Clinical test parameters

Blood samples for haematology and clinical chemistry evaluation were collected from the retro-orbital plexus from animals under light anesthesia induced by CO₂ inhalation after 14 days observation period in the acute oral study and alter 4 weeks of treatment and 14 days of recovery for the repeated dose 4 weeks safety study. EDTA was used as an anticoagulant for haematology samples and sodium citrate was used as an anticoagulant for clinical chemistry. Food was withheld for approximately 18 h before blood collection, and samples were collected early in the working day to reduce biological variation; water was provided *ad libitum.*

Clinical pathology parameters (haematological and clinical biochemistry) were evaluated. Most haematology variables were measured with a Coulter/CELL-DYN 3500 whole blood automated analyzer (Abbott, Chicago, IL). Blood cell smears were observed with an Olympus Microscopy BX41 (Olympus, Tokyo, Japan).

Clinical chemistry parameters were evaluated with a spectrophotometer Konelab PRIME 30 (Thermofisher Scientific Inc. Waltham, MA, USA) and special biochemistry parameters with a clinical chemistry analyzer AU640 (Olympus, Tokyo, Japan). Coagulation parameters were analyzed with a coagulation analyzer Coatron M1 (Teco Medical Instruments, GMBH, Neufahrn, Germany).

### 14.4. Anatomical pathology

All rats were euthanized by CO₂ inhalation and necropsied. The necropsy included a macroscopic examination of the external surface of the body, all orifices, the cranial cavity, the brain and spinal cord, the nasal cavity and paranasal sinuses, and the thoracic, abdominal, and pelvic cavities and viscera. Descriptions of all macroscopic abnormalities were recorded. Samples of the following tissues and organs were collected from all animals at necropsy and fixed in neutral phosphate-buffered 4% formaldehyde solution: adrenal glands, brain, heart, ileum, jejunum, caecum, colon, duodenum, rectum, stomach, oesophagus, trachea, kidneys, liver, lungs, pancreas, spleen, skin, testicles with epididymes, ovaries with oviducts, bone marrow, thymus, thyroid and parathyroid glands, seminal vesicles, urinary bladder and uterus. The organ:body weight ratios were calculated. All organ and tissue samples for histopathological examination were processed, embedded in paraffin, cut at an approximate thickness of 2 to 4 µm, and stained with hematoxylin and eosin. Slides of all organs and tissues listed above were collected from all animals of the control and treated groups.

### 14.5. Isolation of L. salivarius V4II-90 from feces and vaginal swabs samples.

Once the rats were euthanized a sample of colon content and a vaginal swab was collected from each animal. These samples were diluted and inoculated onto MRS-C agar plates. Isolates identified as *L. salivarius* by MALDI-TOF were submitted to pulsed-field gel electrophoresis (PFGE) genotyping (Arroyo *et al*., 2010), and their profiles were compared with that of *L. salivarius* V4II-90.

### 14.6. Bacterial translocation

Bacterial translocation was analysed in blood, liver and spleen. Blood (50 µl) was cultured in de Man, Rogosa, Sharpe (MRS) agar medium and incubated at 37°C during 48 h anaerobically. Tissue samples were homogenized in buffered peptone water (1 g/ml) and 100 µl of the resulting homogenates were cultured on MRS agar as previously mentioned. After 48 h, the plates were checked for the presence of lactobacilli. Positive growth on MRS agar plates was defined by the presence of even a single colony.

### 14.7. Total liver glutathione (GSH) concentration

A portion of 100 mg of liver from each mouse were homogenized in a 7.5% trichloroacetic acid solution and homogenates were centrifuged at 3,000 ×*g* for 10 min at 4°C. Total glutathione concentration was measured in the supernatants using a colorimetric commercial kit (OxisResearch, Portland, OR). Briefly, 40 µl of the homogenates or the standards were added to each well of a microtiter plate, together with 40 ml of a reducing agent (tris(2-carboxyethyl) phosphine in HCI), 40 ml of a chromogen (1-methyl-3-chloro-7-trifluoromethylquinolinium methylsulfate in HCI) and 40 ml of color developer (NaOH). After incubation at room temperature and in the dark for 30 min, optical density was measured at 415 nm using a microplate spectrophotometer (Bio-Rad Laboratories, Hercules CA).

### 14.8. Statistical analysis (toxicity studies)

All data are expressed as means ± standard error of the mean (SEM) of 6 determinations (i.e. 6 males and 6 females). Differences between control and treated groups were evaluated with a one-way analysis of variance (ANOVA) followed by Dunnett's test (1995), and differences were considered significant at P < 0.05.

### 15. Design of a PCR assay for specific and sensitive detection of S. agalactiae in the biological samples.

### 15.1. Primers design

In order to design GBS species-specific primers, 16S rRNA gene sequences from various bacterial species available in the Genbank database were analyzed and compared using the programs *Emma* and *Showalign,* included in the free open source software analysis EMBOSS (*The European Molecular Biology Open Software Suite*). Specific amplification of a 148 bp fragment of the 16S rRNA gene of *S. agalactiae* was achieved.

The bacterial species in which the 16S rRNA gene sequence from was analyzed and compared were the following: *L. plantarum* (EF85922122), *Ped. Pentosaceus* (CP000422), *L. fermentum* (EF460496), *W. minor* (NR040809), *S. epidermidis* (D83363), *S. aureus* (D83357), *S. xylosus* (D83374), *E. faecalis* (EF120452), *E. faecium* (DQ672262), *S. mitis* (AY281078), *S. parasaguinis* (AY281087), S. *oralis* (DQ232537), *S. oralis* (AY281079), *S. infantis* (AY485603), *S. peroris* (AB008314), *S. salivarius* (AY188352), *S. bovis* (AB002481), *S. agalactiae* (AE00948), *S. agalactiae* (AB596948), *L. lactis* (CP002365), *L. mesenteroides* (CP000414), *B. bifidum* (CP001840) and *B. vulgatus* (NC009614).

### 15.2. Conventional and Real-Time PCR assays

All reactions were set up using the CFX Connect and CFX96 Touch™ Real-Time PCR Detection Systems (Bio-Rad Laboratories, Inc, Hercules, CA, USA). The reaction volume for conventional PCR was 25 µL, and 10 µL for real-time PCR amplification. Each run contained a negative control (deionized sterile water) and a positive control (100 or 25 ng of *S. agalactiae* DSMZ 2134).

### 15.3. Specificity

The specificity of the primers designed was tested using DNA obtained from a panel of 6 *S. agalactiae* strains, 10 strains of other streptococcal species, and 21 strains of species belonging to related genera. All strains were analyzed in duplicate.

In a real-time assay the determination of a PCR positive result and the specificity of the reaction, in the absence of probes, were made based on the presence of a melting curve. The melting temperature (Tm), which is specific for each amplicon, depends on various factors including the amplicon length and the nucleotide sequence.

### 15.4. Detection limit

To check the detection limit of this PCR approach, the DNA extracted from pure culture of *S. agalactiae* DSMZ 2134, obtained from the German Collection of Microorganism and Cell Cultures (DSMZ), was used to prepare 10-fold dilution series with a bacterial population ranging from 2 to 9 log₁₀ colony-forming units, as determined by plate counts.

### 15.5. Efficiency

The DNA extracted from vagino-rectal swab samples provided by female volunteers was analyzed in order to evaluate the applicability of the conventional and real-time PCR assay to the detection of *S. agalactiae* colonization in women. Both PCR approaches were compared with a conventional culture approach and GBS identification by MALDI-TOF, as explained above. All samples were analyzed in duplicate.

In the real-time PCR assay the presence or absence of GBS DNA in a sample was defined using a cut-off Ct value equal to 38.64 (the mean value less twice the standard deviation of the non-target species and non-template controls). *Streptococcus agalactiae* DNA was considered present if a Ct value of less than 38.64 was obtained.

### 16. Efficacy of L. salivarius V4II-90 to eradicate GBS from the intestinal and vaginal tracts of pregnant women: clinical trial

### 16.1. Design of the trial

A total of 87 pregnant women (69 rectal and/or vaginal GBS-positive women; 18 rectal and vaginal GBS-negative women at the start of the intervention), aged 25-36, participated in this study. All met the following criteria: a normal pregnancy and a healthy status. Women ingesting probiotic supplements or receiving antibiotic treatment in the previous 30 days were excluded. All volunteers gave written informed consent to the protocol (10/017-E), which had been approved by the Ethical Committee of Clinical Research of Hospital Clínico San Carlos Madrid (Spain).

Volunteers were distributed into 5 groups (3 probiotic and 2 placebo groups). All the volunteers in any of the probiotic groups (*n* = 55) were GBS-positive and consumed a daily sachet with ∼50 mg of freeze-dried probiotic (∼9 log₁₀ cfu of *L. salivarius* V4II-90). Probiotic subgroups 1 (*n* = 25), 2 (*n* = 16) and 3 (*n* = 14) started the intervention at pregnancy weeks 26, 20-24 or 30-34, respectively. Placebo subgroup 1 (*n*=14) included GBS-positive women (pregnancy week ranging from 19 to 30) that were going to receive intrapartum antibiotic prophylaxis (IAP) because they had a previous baby that suffered a GBS sepsis. Placebo subgroup 2 (*n*=18) included GBS-negative women (pregnancy week ranging from 14 to 26). Women in both placebo subgroups received a daily sachet containing 50 mg of the excipient used to vehiculate the probiotic strain. In all cases, the intervention lasted from the start of the intervention to week 38, when recto-vaginal GBS screening was performed. Probiotic- and excipient-containing sachets were kept at 4°C throughout the study. All volunteers were provided with diaries to record compliance with study product intake. Minimum compliance rate (% of the total treatment doses) was set at 86%.

### 16.2. Collection and GBS analysis of the samples

Rectal and vaginal exudates samples were collected periodically during the trial. They were serially diluted and plated on Granada (Biomerieux; isolation of haemolytic GBS, which appear as orange colonies), and CHROMagar StrepB (CHROMagar; for isolation of haemolytic and non- haemolytic GBS, which appear as purple colonies) agar plates. Correct taxonomic assignment was confirmed by MALDI-TOF and latex agglutination analyses as described previously.

Parallel, and to avoid sensitivity-related problems, two strategies were used: (a) DNA was extracted from the samples and submitted to the PCR assays described above; (b) a subset of samples was submitted to a GBS enrichment step in Todd-Hewitt broth (Oxoid). After 24 h at 37°C, the broth cultures were spread on CHROMagar StrepB agar plates.

### 16.3. Statistical analysis

Microbiological data were recorded as CFU/mL, and transformed to logarithmic values before statistical analysis. Two-way ANOVA was used to investigate the effect of individual (woman) and sampling time on the semiquantitative *S. agalactiae* counts in vaginal swabs. Statistical significance was set at P<0.05. Statgraphics Centurion XVI version 17.0.16 (Statpoint Technologies Inc, Warrenton, Virginia) was used to carry out statistical analyses.

### Example 1. Assessment of the vaginal microbiota of GBS-positive and GBS-negative fertile women (non-pregnant and pregnant women)

In all cases, bacterial growth was detected when the samples were inoculated on MRS (2.70-8.08 log₁₀ cfu; mean 5.36 log₁₀ cfu); CNA (3.00-7.92 log₁₀ cfu; mean 5.13 log₁₀ cfu) and GAR (2.70-8.10 log₁₀ cfu; mean 5.24 log₁₀ cfu) agar plates. Globally, similar bacterial groups grew in the three media (data not shown). Growth on MCK, SDC or Mycoplasma plates was only detected in a few percentage of samples (from 0% in Mycoplasma plates to ∼40% in SDC plates).

*S. agalactiae* could be isolated from both non-pregnant (∼25%) and pregnant (∼20%) women. *Candida albicans* and other yeasts were isolated from, approximately, 7 and 36% of the non-pregnant and pregnant women, respectively. In both groups, *Lactobacillus* were the dominant genus since it was detected in 92-93% of the participating women.

In relation to the samples provided by non-pregnant women, a total of 433 isolates (including, at least, one representative of each colony and cell morphology) were submitted to taxonomical analyses. The genus *Lactobacillus* was the dominant one (28% of the total isolates), followed by *Staphylococcus, Enterococcus, Corynebacterium* and *Streptococcus* (Table 1). Among the isolates, the main species were *Lactobacillus crispatus* (23%), *Lactobacillus gasseri* (24%), *L. salivarius* (21%), *Lactobacillus vaginalis* (12%), *Lactobacillus plantarum* (13%), *Lactobacillus coleohominis* (5%) and *Lactobacillus jensenii* (2%).

From the samples provided by pregnant women, 120 isolates were submitted to taxonomical analyses. Again, the genus *Lactobacillus* was the dominant one (17% of the total isolates), followed by *Staphylococcus, Streptococcus,* yeasts and *Enterococcus,* (Table 1). Among the isolates, the main species were *L. gasseri* (41%), *L. casei* (19%), *L. salivarius* (16%), *Lactobacillus fermentum* (8%), *L. vaginalis (6%), Lactobacillus reuteri* (5%) and *L. jensenii (5%).*

### Example 2.Preliminary selection of the Lactobacillus strains:

As stated above, the 89 different lactobacilli strains isolated in this study belonged to the following species: *L. salivarius, L. gasseri, L. vaginalis, L. jensenii, L. crispatus, L. plantarum, L. coleohominis, L. casei, L. reuteri* and *L. fermentum.* Among them, a few were selected on the basis of the following criteria: (1) absence of *S. agalactiae, Gardnella vaginalis, Candida* spp., *Ureaplasma* spp. and *Mycoplasma* spp. in the vaginal samples from which the lactobacilli were originally isolated; (2) Qualified Presumption of Safety (QPS) status (European Authority of Food Safety, EFSA) of the *Lactobacillus* species, since there was the intention of using them as food supplements; and (3) ability of the strain to grew rapidly in MRS broth under aerobic conditions (≥1 ×10⁶ cfu/mL after 16 h at 37°C). Only 10 strains (V3III-1, V4II-90, V7II-1, V7II-62, V7IV-1, V7IV 60, V8III-62, V111-60, V11III-60 y V11IV-60) met all the criteria and all of them belonged to the same species (*L. salivarius*). These strains were those selected for further characterization.

### Example 3. Antimicrobial activity and production of potential antimicrobial compounds:

Initially, the antimicrobial activity of the 10 selected lactobacilli against the *S. agalactiae* strains was determined by an overlay method. Clear inhibition zones (ranging from 2 to 20 mm) were observed around the lactobacilli streaks.

In relation to the antimicrobials compounds that may be responsible for such activity, the concentration of L- and D-lactic acid and the pH of the supernatants obtained from MRS cultures of the lactobacilli are shown in Table 2. The global concentration of L-lactic acid was similar (-10 mg/mL) in all the supernatants. In contrast, D-lactic acid was not detected in the supernatants of the tested strains. In addition, all the strains acidified the MRS-broth medium to a final pH of ∼4 after 16 h of incubation; among them, *L. salivarius* V7IV-1 showed the highest acidifying capacity (final pH of 3.8).

No bacteriocin-like activity could be detected against the tested *S. agalactiae* strains. Two strains (L. *salivarius* V4II-90 and V7IV-1) were able to produce hydrogen peroxide (7.29 µg/mL ± 0.69 and 7.46 µg/mL ± 0.58, respectively) (Table 2).

### Example 4. Co-aggregation between the lactobacilli and the S. agalactiae strains:

The capacity of the lactobacilli strains to form large, well defined co-aggregates with *S. agalactiae* was strain-dependent. Strains V3III-1, V7IV-60 and V11IV-60 coaggregated with 5 *S. agalactiae* strains, strains V8III-62, V11I-60 and V11III-60 with 7, strain V7II-62 with 9, and strains V4II-90, V7II-1 and V7IV-1 with 10 (Figure 1).

### Example 5. Broth co-cultures of the lactobacilli and the S. agalactiae strains:

The ability of the lactobacilli strains to interfere or inhibit the growth of four S. *agalactiae* strains was evaluated using MRS broth co-cultures. Co-cultures with S. *agalactiae* seemed not to affect the growth of any of the *L. salivarius* strains (Table 3). In contrast, most of the *L. salivarius* strains were able to interfere at a higher or lower degree with the growth of the different *S. agalactiae* strains included in this assay. Among them, *L. salivarius* V4II-90 showed the highest ability to inhibit the growth of *S. agalactiae* since the presence of two of the four *S. agalactiae* strains was not detectable in the co-cultures and the concentration of the other two showed a ∼2.5 log₁₀ decrease after an incubation period of only 6 h at 37°C (Table 3). Interestingly, no viable streptococci could be detected when the in the co-cultures were incubated for 24 h (Table 3).

### Example 6. Adherence assays to intestinal and vaginal epithelial cells:

In this study, the lactobacilli strains tested were strongly adhesive to both Caco-2 and HT-29 cells, with the exception of the negative control strain (*L*. *casei* imunitass) which showed a low adhesive potential (Table 4). In addition, all showed adhesion to vaginal epithelial cells. Among the *L. salivarius* strains, *L. salivarius* V4II-90 globally displayed the highest ability to adhere to both intestinal and vaginal epithelial cells (Table 4).

### Example 7. Adherence to and/or degradation of mucin:

The lactobacilli strains tested showed a variable ability to adhere to porcine mucin (Table 5). *L. reuteri* CR20 (positive control strain) was the strain that showed the highest adherence ability followed by *L. salivarius* V4II-90 and *L. salivarius* V7IV-1 (Table 5). None of the strains were able to degrade gastric mucin *in vitro.*

### Example 8. Survival after transit through an in vitro gastrointestinal model:

The viability of the strains after exposition to conditions simulating those found in the gastrointestinal tract varied from ∼64% (*L. reuteri* CR20, *L. salivarius* V4II-90) to 30% (*L. salivarius* V3III-1) (Table 6).

### Example 9. Antibiotic susceptibility:

The MIC values of the lactobacilli strains for 16antibiotics assayed are shown in Table 7.All the strains were sensitive to most of the antibiotics tested, including those considered clinically relevant antibiotics such as, gentamycin, tetracycline, clindamycin, chloramphenicol, and ampicillin, showing MICs equal to, or lower than, the breakpoints defined by EFSA (EFSA, 2012). All the strains were resistant to vancomycin and kanamycin, which is an intrinsic property of the *L. salivarius* at the species level.

### Example 10. Prophage induction:

None of the supernatants obtained from the *L. salivarius* strains generated phage-related inhibition halos or plaques of lysis on lawns of any of indicator strains tested in this study.

### Example 11. Production of biogenic amines:

The *L. salivarius* strains neither produced biogenic amines nor harboured the genes required for the biosynthesis of this type of compounds.

### Example 12. In vivo safety assessment of L. salivarius V4II-90:

### 12.1. Acute oral toxicity in rats:

No abnormal clinical signs, behavioural changes, body weight changes, macroscopic findings, or organ weight changes were observed. All animals survived the 2-week observation period. There were no statistical differences in body weights among groups. Similarly, no statistically significant differences in body weight gain, food and water consumption were noted. Body weight, daily body weight gain, food and water consumption thus were unaffected by the treatment (single oral dose of 1 × 10¹⁰ cfu of *L. salivarius* V4II-90).

The haematological and clinical chemistry parameters assessed 2 weeks after administration of the strain as a single oral dose of 1 × 10¹⁰ cfu were not significantly different compared with those of controls (Tables 8 and 9). No treatment-related changes were noted.

There were no statistical differences in organ weight or tissue: body weight ratios related to the test strain (data not shown). The *L. salivarius* V4II-90 preparation was not associated with any incidence of macroscopic and microscopic changes. No treatment-related histopathological changes were observed 2 weeks after administration of the strain as a single oral dose of 1 × 10¹⁰ cfu. Therefore, *L*. *salivarius* V4II-90 has a low order of acute toxicity and the oral lethal dose (LD50) for male and female rats is higher than 1 × 10¹⁰ cfu.

### 12.2. Repeated dose (4 weeks) oral toxicity in rats:

No mortality was observed. No treatment-related changes in the general condition and external appearance were observed in male and female rats treated with 1 × 10⁹cfu *of L. salivarius* V4II-90 daily dose. The development of the animals during the experimental period corresponded to their species and age. There was no significant difference in body weight or body weight gain among groups treated with *L. salivarius* V4II-90 in comparison to the control groups at any time point of the experimental period. All *L. salivarius* V4II-90-treated groups consumed similar amounts of food and water (data not shown) to that of the corresponding control groups.

All haematology data were within normal limits and differences between groups were no observed (Table 10). Clinical chemistry data showed no treatment-related alterations at the end of 4-weeks treatment period (Table 11). Individual values and group mean values were within the physiologic ranges. After 14 days without treatment to detect delayed occurrence of potential toxic effects, there were no treatment-related changes neither in haematological nor in clinical test parameters (Tables 10 and 11; satellite control group or Group 5 and satellite treated group or Group 6).

The necropsy performed on day 29 after the last dose of *L. salivarius* V4II-90 (Group 4) and on day 42 after 14 days without any treatment (Group 6) did not reveal any gross pathological changes or any differences in organ weights in comparison to the corresponding control groups. Mean organ weights and rate body weight:organ weights are presented in Table 12. After 4-weeks of treatment, there were no histopathological findings in the organs examined considered to be treatment related in male and female rats (data not shown). There were also no treatment-related histopathological findings in the satellite treated group (Group 6) (data not shown).

The no-observed-adverse-effect level in this repeated dose (4 weeks) oral toxicity study was the dose tested, i.e. 1 × 10⁹ cfu of *L. salivarius* V4II-90.

### 12.3. Total liver glutathione (GSH) concentration and potential bacteremia:

In order to determine changes in the antioxidant defense because of the probiotic treatment, liver GSH concentration was determined. No significant differences in liver GSH concentration were observed between control and treated groups (9.54±1.21 vs 9.37±1.39 mmol/g, P>0.1). This indicates that treatment with *L. salivarius* V4II-90 did not cause oxidative stress to rats and is consistent with the absence of bacteremia since no lactobacilli could be isolated from blood, liver or spleen of the rats. It suggests that the tested strain do not cause either a local or a systemic infection in rats.

### 12.4. Isolation of L. salivarius V4II-90 from feces and vaginal swabs samples:

*L. salivarius* V4II-90could be isolated from colonic material and vaginal swabs samples of all the treated animals (probiotic groups) at the end of the treatment. The concentration oscillated between 5 × 10⁵ and 7 × 10⁸ cfu/g of colonic material, and between 3 × 10³ and 23 × 10⁶ cfu/g in the vaginal swabs. The strain could not be detected in any sample from the placebo group.

### Example 13. Design of a PCR assay for specific and sensitive detection of S. agalactiae in the biological samples:

On the basis of sequence comparison using programs *Emma* and *Showalign,* primers *S.agalacFW1* (5'-GTTTGGTGTTTACACTAGA-'3) (SEQ ID NO: 4) and *S.agalacRV1* (5'-CAATTGCTCCTTTTAAATAACT-'3) (SEQ ID NO: 5) were designed for the specific amplification of a theoretical 148 bp fragment of the 16S rRNA gene of S. *agalactiae.*

In conventional PCR, the GBS-specific primer pair (*S.agalacFW1* and *S.agalacRV1*) amplified a single 148 bp amplicon from *S. agalactiae* DNA. Cross-reactivity was not obtained with DNA from any other species analyzed (Figure 2).

The real-time specific system amplified a 148 bp fragment, with a melting temperature of 80.00 from all the *S. agalactiae* samples analyzed. No homologous product was amplified from any other bacterial DNA tested. Threshold cycle (Ct) values between 16.72 and 20.87 were obtained from *S. agalactiae* DNA. The Ct values measured for DNA extracted from non-target species were 39.82 ± 0.59 (Table 13). Under our experimental conditions, a cut-off value was establish as follows: Ct values above that corresponding to the mean Ct value of all the non-target species and non-template controls minus twice their standard deviation (Ct > 38.64) were considered negative for the presence of target DNA. In relation to the detection limit of this PCR approach, it was observed that the lower percentage of target DNA, the higher Ct values obtained in the real-time PCR with the species-specific primers. The detection limit of this assay was lower than 0.005 ng of target DNA, which corresponds to Ct value of 34.34 (Figure 3, Table 14).

When real biological samples were tested, there was an excellent agreement between conventional PCR results and those obtained by culture-based methods (Table 15). MALDI-TOF and conventional (qualitative) PCR identification were discordant for only 1 sample. In addition, two samples that were positive by conventional PCR provided a negative result by real-time PCR. Globally, the results obtained indicate that the PCR techniques developed in this study have proven to have a high specificity and sensitivity, and, therefore, they constitute useful GBS screening methods.

### Example 14. Efficacy of L. salivarius V4II-90 to eradicate GBS from the intestinal and vaginal tracts of pregnant women: clinical trial:

At the inclusion in the study, GBS was detected in both rectal and vaginal swabs obtained from 39 women, out of a total of 57 participating women, while the rest of women (n = 18) were GBS-negative (Table 16). This last group of GBS-negative women, which did not take a *L. salivarius* strain (10⁹ cfu/daily), also had negative GBS cultures from rectal and vaginal swabs taken regularly at 28, 32 and 36-38 weeks. A group of GBS-positive women at the start of the study (n = 14) did not receive probiotic and the routine screening results for vaginal and rectal GBS at 28, 32 and 36-38 weeks were found to be all positive (Table 16; Figure 4).

Significantly, the group of GBS-positive women that started using the probiotic (10⁹ cfu/daily) since they were enrolled in this study (from 26 weeks) also tested positive for GBS at 28 weeks, but an increasing number of GBS-negative results appeared in the successive swabs collected until delivery (Table 16; Figure 4). At 30 weeks, culture of rectal swabs taken from four women of this group rendered a negative result and the number of these samples increased to 18 (72% of the participants) at 38 weeks. Similar results were obtained culturing vaginal swabs obtained from this group, although the proportion of women testing negative for GBS were always slightly higher when analyzing the rectal swabs than in vaginal swabs (Table 16; Figure 4).

A more detailed analysis revealed that the GBS absence in rectal and vaginal swabs was detected at the same sampling time in 6 participants among women with GBS-negative samples (n = 19) (one woman at 35 weeks and five women at 32 weeks). The detection of GBS absence was most frequently found for the first time in rectal swabs (11 women) than in vaginal swabs (2 women) (Table 17).

The estimation of the concentration of GBS in vaginal swabs taken regularly up to the delivery from all participants is shown in Figure 5. There were no significant changes in both GBS-negative women (n = 18) and GBS-positive women (n = 14) without oral administration *of L. salivarius* V4II-90 regarding the semiquantitative estimation of GBS. However, the number of vaginal swabs where the GBS could not be detected increased in successive sampling times in the group that initially tested positive for GBS taking10⁹ cfu *of L. salivarius* V4II-90 (n = 25). Furthermore, the change in mean bacterial counts for *S. agalactiae* in those women that tested positive and took the *L. salivarius* strain depended both on women and sampling time, but there was no interaction between these two factors (two-way ANOVA; P<0.001). The mean value for *S. agalactiae* counts decreased significantly with the administration time of *L. salivarius* V4II-90 (Figure 6) from a mean value of 5.14 cfu/mL at 26 weeks (n=25) to 3.80 cfu/mL at 38 weeks (n=9) (Figure 6).

No adverse effects arising from the intake of *L. salivarius* V4II-90 were reported by any of the women that participated in this study.

### CONCLUSIONS OF THE EXAMPLES:

In the present invention, all the vaginal isolates (from either pregnant or non-pregnant healthy women) that fulfilled the initial selection criteria belonged to the same species: *L. salivarius.*

In the present invention none of the selected *L. salivarius* strains displayed bacteriocin-like activity against *S. agalactiae* strains. Therefore, the antimicrobial activity that the selected *L. salivarius* strains exhibited against *S. agalactiae* must be related with the production of other antimicrobial compounds, such as organic acids. In our study, *L. salivarius* V4II-90 (the strain that showed the highest anti-GBS activity) produced high amounts of lactic acid and, in addition, was able to produce peroxide hydrogen.

The ability to adhere to intestinal or vaginal epithelial cells or to mucin, and to co-aggregate with potential pathogens constitutes one of the main mechanisms for preventing their adhesion and colonization of mucosal surfaces. Globally, *L. salivarius* V4II-90 showed the best combination of adherence to epithelial cells, co-aggregation with *S. agalactiae* and inhibition of *S. agalactiae* strains in broth co-cultures. This strain showed a high survival rate during transit through an *in vitro* gastrointestinal model, similar to those obtained with other *L. salivarius* using the same model (Martin *et al.,* 2006; Martin *et al.,* 2009). Survival of lactobacilli when exposed to conditions found in the gastrointestinal tract seems to be a critical prerequisite for a probiotic strain when use as a food supplement is pursued, as it was the case.

One of the most important criteria for the selection of probiotic strains is the assessment of their safety, particularly to the target population. In this work, no adverse effect was reported by any of the women participated in the clinical trial and adscribed to any of the three probiotic groups (thus, receiving *L. salivarius* V4II-90 at 9 log10 cfu daily for several weeks). Safety must be assessed in a strain by strain manner since, although rare, adverse effects due to the consumption of lactobacilli strains have been reported.

The *L. salivarius* strains included in this study were very susceptible to most of the antimicrobials tested. In fact, their MICs were lower than the cut-offs established for lactobacilli to seven out of the eight antibiotics required for this species by the European Food Safety Authority (EFSA, 2012). The only exception was kanamycin. Resistance of lactobacilli to kanamycin and other aminoglycosides (such as neomycin or streptomycin) has been repeatedly reported, and this is thought to be a *L. salivarius* species-specific trait due to the lack of cytochrome-mediated transport of this class of antibiotics; the *L. salivarius* strains were also resistant to vancomycin but assessment of vancomycin sensitivity is not required by EFSA in the case of homofermentative lactobacilli (including *L. salivarius)* since they are intrinsically resistant to this antibiotic probably due to the presence of D-Ala-D-lactate in their peptidoglycan structure. As recognized by EFSA (2012), these types of intrinsic resistances do not represent a human health risk. Therefore, *L. salivarius* V4II-90 and the other strains evaluated in this study can be considered as safe from this point of view.

Lactobacilli are among the Gram-positive bacteria with potential to produce biogenic amines; since these substances can cause several toxicological problems and/or may act as potential precursors of carcinogenic nitrosamines, nevertheless, the screened *L. salivarius* strains neither produced histamine, tyramine, putrescine or cadaverine nor harboured the gene determinants required for their biosynthesis. Additionally, they were unable to degrade gastric mucin *in vitro.* Therefore the strain of the present invention does not cause toxicological problems.

### TABLES

**Table 1. Main genera and species isolated from the vaginal samples provided by the women that participated in the study.**

| **Non-pregnant women** | | | **Pregnant women** | | |
|---|---|---|---|---|---|
| Genus/groups | % | Main species | Genus/groups | %) | Main species |
| *Lactobacillus* | 28 | *L. crispatus* (23%) | *Lactobacillus* | 18 | *L. gasseri* (41%) |
| | | *L. gasseri* (24%) | | | *L. casei* (19%) |
| | | *L. salivarius* (21%) | | | *L. salivarius* (16%) |
| | | *L. vaginalis* (12%) | | | *L. fermentum* (8%) |
| | | *L. plantarum* (13%) | | | *L. vaginalis* (6%) |
| | | *L. coleohominis* (5%) | | | *L. reuteri* (5%) |
| | | *L. jensenii* (2%) | | | *L. jensenii* (5%) |
| *Staphylococcus* | 17 | *S. epidermidis* | *Staphylococcus* | 17 | *S. epidermidis* |
| | | *S. hominis* | | | *S. hominis* |
| | | *S. aureus* | | | *S. aureus* |
| *Enterococcus* | 11 | *E. faecalis* | *Streptococcus* | 10 | *S. agalactiae* (60%) |
| *Corynebacterium* | 7 | *C. pseudogenitalium* | Yeasts | 10 | *C. albicans* |
| | | *C. afermentans* | | | *C. glabrata,* |
| | | *C. aurimucosum* | | | *C. parapsilosis* |
| *Streptococcus* | 4 | *S. agalactiae* (75%) | *Enterococcus* | 6 | *E. faecalis* |
| | | *S. sanguinis* | | | |
| | | *S. anginosus* | | | |
| Others | 12 | *Streptomyces albus* | Others | 12 | *Bifidobacterium* |
| | | *Dermabacter hominis* | | | spp.(30%) |
| | | *Propionibacterium avidum* | | | *Corynebacterium* |
| | | *Aerococcus urinae* | | | spp. (10%) |
| | | *Dermacoccus* | | | |
| | | *nishinomiyaensis* | | | |
| | | *Actinomyces neuii* | | | |
| | | *Roseomonas mucosa* | | | |
| | | *Facklamia spp.* | | | |
| | | *Arthrobacter cumminsii* | | | |
| | | *Brevibacterium sp* | | | |
| | | *Helcobacilius massiliensis* | | | |
| | | *E coli* | | | |
| | | Yeasts (*C. albicans, C. glabrata, C. parapsilosis*) | | | |
| Non-identified | 21 | | Non-identified | 27 | |

| | | | | | |
|---|---|---|---|---|---|
| "%", percentage | | | | | |

**Table 2. pH and concentrations of L- and D-lactic acid (mg/mL; mean ± SD), and hydrogen peroxide (mg/mL; mean ± SD) in the supernatants obtained from MRS cultures of the lactobacilli (n=4).**

| Strain | pH | L -lactic acid | D-lactic acid | Hydrogen peroxide |
|---|---|---|---|---|
| *L. salivarius*V3III-1 | 4.00 | 9.66 ± 0.57 | Nd | Nd |
| *L. salivarius*V4II-90 | 4.01 | 10.03 ± 0.60 | Nd | 7.29 ± 0.69 |
| *L. salivarius*V7II-1 | 4.02 | 9.82 ± 0.69 | Nd | Nd |
| *L. salivarius*V7II-62 | 4.01 | 9.76 ± 0.54 | Nd | Nd |
| *L. salivarius*V7IV-1 | 3.85 | 10.47 ± 0.58 | Nd | 7.46 ± 0.58 |
| *L. salivarius*V7IV-60 | 4.02 | 9.72 ± 0.63 | Nd | Nd |
| *L. salivarius*V8III-62 | 4.04 | 9.91 ± 0.55 | Nd | Nd |
| *L. salivarius*V11I-60 | 4.03 | 9.84 ± 0.43 | Nd | Nd |
| *L. salivarius*V11III-60 | 4.07 | 9.61 ± 0.47 | Nd | Nd |
| *L. salivarius*V11IV-60 | 4.03 | 10.02 ± 0.62 | Nd | Nd |
| *L. salivarius* CECT 5713 | 3.93 | 10.26 ± 0.62 | Nd | - |

The initial pH value of MRS broth was 6.2. ND: not detectable.

**Table 3. Bacterial counts of the S. agalactiae strains when co-cultured with the L. salivarius strains in MRS broth for 0, 6 and 24 h at 37°C.**

| | | | | |
|---|---|---|---|---|
| *L. salivarius* (strain) | *S. agalactiae* | 0 h | 6 h | 24 h |
| V3III-1 | RC5 | 7.10 | 6.44 | Nd |
| | RC6 | 7.24 | 7.04 | Nd |
| | V2I-80 | 7.10 | 7.04 | Nd |
| | V14I-63 | 7.27 | 7.10 | Nd |
| V4II-90 | RC5 | 7.04 | 4.48 | Nd |
| | RC6 | 7.23 | Nd | Nd |
| | V2I-80 | 7.10 | 4.70 | Nd |
| | V14I-63 | 7.34 | Nd | Nd |
| V7II-1 | RC5 | 7.15 | 7.27 | Nd |
| | RC6 | 7.15 | 6.70 | Nd |
| | V2I-80 | 7.04 | 7.10 | Nd |
| | V14I-63 | 7.35 | 5.65 | Nd |
| V7II-62 | RC5 | 7.24 | 7.04 | Nd |
| | RC6 | 6.98 | 7.49 | Nd |
| | V2I-80 | 7.35 | 7.92 | Nd |
| | V14I-63 | 7.10 | 6.93 | Nd |
| V7IV-1 | RC5 | 7.32 | 7.58 | Nd |
| | RC6 | 7.34 | 6.90 | Nd |
| | V2I-80 | 7.15 | 7.38 | Nd |
| | V14I-63 | 7.23 | 6.04 | Nd |
| V7IV-60 | RC5 | 7.24 | 7.32 | Nd |
| | RC6 | 7.32 | 8.06 | Nd |
| | V2I-80 | 7.04 | 7.15 | Nd |
| | V14I-63 | 7.35 | 8.34 | Nd |
| V8III-62 | RC5 | 7.15 | 7.90 | Nd |
| | RC6 | 7.34 | 7.23 | Nd |
| | V2I-80 | 7.24 | 6.90 | Nd |
| | V14I-63 | 7.20 | 8.77 | Nd |
| V11I-60 | RC5 | 7.31 | 7.44 | Nd |
| | RC6 | 7.01 | 6.94 | Nd |
| | V2I-80 | 7.23 | 7.07 | Nd |
| | V14I-63 | 6.93 | 6.60 | Nd |
| V11 III-60 | RC5 | 7.27 | 6.44 | Nd |
| | RC6 | 6.95 | 6.88 | Nd |
| | V2I-80 | 7.28 | 6.52 | Nd |
| | V14I-63 | 7.37 | 6.85 | Nd |
| V11IV-60 | RC5 | 7.26 | 6.74 | Nd |
| | RC6 | 7.42 | 6.60 | Nd |
| | V2I-80 | 7.10 | 6.60 | Nd |
| | V14I-63 | 7.06 | 5.32 | Nd |
| Control cultures | RC5 | 7.20 | 9.32 | 9.34 |
| (no *L. salivarius* strain) | RC6 | 7.31 | 9.20 | 9.27 |
| | V2I-80 | 7.04 | 9.15 | 9.23 |
| | V14I-63 | 7.10 | 9.02 | 9.15 |
| Nd: *S*. *agalactiae* was not detected. | | | | |

**Table 4. Ability of the lactobacilli to adhere to HT-29, Caco-2 and vaginal epithelial cells.**

| Strain | HT-29 | Caco-2 | Vaginal cells |
|---|---|---|---|
| *L. salivarius*V3III-1 | 877.3 ± 303.2 | 259.1 ± 67.1 | + |
| *L. salivarius*V4II-90 | 905.2 ± 297.0 | 345.1 ± 72.8 | +++ |
| *L. salivarius*V7II-1 | 900.5 ± 336.2 | 297.8 ± 84.5 | ++ |
| *L. salivarius*V7II-62 | 911.7 ± 250.9 | 321.5 ± 80.2 | ++ |
| *L. salivarius*V7IV-1 | 884.0 ± 226.3 | 252.3 ± 67.1 | ++ |
| *L. salivarius*V7IV-60 | 799.7 ± 210.1 | 255.9 ± 60.3 | ++ |
| *L. salivarius*V8III-62 | 623.4 ± 200.2 | 108.7 ± 24.3 | + |
| *L. salivarius*V11I-60 | 593.2 ± 191.5 | 121.6 ± 22.0 | + |
| *L. salivarius*V11III-60 | 612.4 ± 188.2 | 153.2 ± 26.7 | + |
| *L. salivarius*V11IV-60 | 601.6 ± 172.0 | 159.5 ± 23.4 | + |
| *L. rhamnosus* GG | 912.4 ± 345.0 | 371.5 ± 67.8 | Nd |
| *L. casei* imunitass | 127.4 ± 20.9 | 16.7 ± 7.3 | Nd |

The adherent lactobacilli in 20 random microscopic fields were counted for each test (n=4). Nd, not determined.

**Table 5. Ability of the lactobacilli to adhere to porcine mucin.**

| Strain | Adhesion^{a} |
|---|---|
| *L. salivarius*V3III-1 | 9.3 ± 2.0 |
| *L. salivarius*V4II-90 | 10.9 ± 1.8 |
| *L. salivarius*V7II-1 | 8.9 ± 1.9 |
| *L. salivarius*V7II-62 | 9.0 ± 1.6 |
| *L. salivarius*V7IV-1 | 8.5 ± 1.2 |
| *L. salivarius*V7IV-60 | 9.6 ± 1.7 |
| *L. salivarius*V8III-62 | 3.3 ± 0.7 |
| *L. salivarius*V11I-60 | 2.9 ± 0.8 |
| *L. salivarius*V11III-60 | 2.4 ± 1.0 |
| *L. salivarius*V11IV-60 | 3.4 ± 0.8 |
| *L. reuteri* CR20 | 11.8 ± 1.9 |

| | |
|---|---|
| ^{a}Values expressed as the percentage of the fluorescence retained in the wells after the washing steps of the assay. | |

**Table 6. Percentage (%) of initial lactobacilli (10⁹ cfu/mL) that survived to conditions simulating those of the gastrointestinal tract.**

| | Gastric-emptying fraction^{a} | | | | |
|---|---|---|---|---|---|
| Strain | 20 min | 40 min | 60 min | 80 min | % Total |
| *L. salivarius*V3III-1 | 9.4 ± 0.4 | 12.1 ± 0.5 | 5.0 ± 0.3 | 3.7 ± 0.1 | 30.2 |
| *L. salivarius*V4II-90 | 16.9 ± 0.6 | 21.3 ± 1.7 | 16.5 ± 0.9 | 9.3 ± 0.5 | 64.3 |
| *L. salivarius*V7II-1 | 14.8 ± 0.4 | 23.7 ± 2.4 | 14.3 ± 1.4 | 7.0 ± 0.4 | 59.8 |
| *L. salivarius*V7II-62 | 14.3 ± 2.0 | 12.0 ± 1.3 | 11.5 ± 0.7 | 12.7 ± 1.5 | 50.5 |
| *L. salivarius*V7IV-1 | 13.6 ± 0.8 | 13.5 ± 1.2 | 11.9 ± 1.3 | 9.1 ± 1.4 | 48.1 |
| *L. salivarius*V7IV-60 | 12.8 ± 1.9 | 16.3 ± 1.5 | 10.7 ± 1.2 | 13.5 ± 1.6 | 53.3 |
| *L. salivarius*V8III-62 | 7.5 ± 0.6 | 12.4 ± 1.0 | 10.8 ± 1.2 | 10.6 ± 1.4 | 41.3 |
| *L. salivarius*V11I-60 | 8.2 ± 0.7 | 11.7 ± 1.4 | 11.3 ± 1.1 | 9.6 ± 1.3 | 40.8 |
| *L. salivarius*V11III-60 | 7.9 ± 0.8 | 12.0 ± 1.2 | 10.2 ± 1.3 | 11.0 ± 1.6 | 41.1 |
| *L. salivarius*V11IV-60 | 8.5 ± 0.7 | 12.7 ± 1.3 | 10.9 ± 0.9 | 10.2 ± 1.1 | 42.3 |
| *L. salivarius*CELA2 | 15.4 ± 1.6 | 25.8 ± 2.9 | 17.0 ± 2.2 | 6.2 ± 0.4 | 64.4 |

| | | | | | |
|---|---|---|---|---|---|
| ^{a}Mean ± SD. The different fractions were taken from the gastric-like compartment at 0, 20, 40, 60, and 80 min and later submitted to the intestinal-like secretion. | | | | | |

**Table 7. Minimal inhibitory concentration (MIC, mg/ml) values of 16 antibiotics^{a} to the L. salivarius strains.**

| Antibiotic | | | | | | | | | | | | | | | | |
|---|---|---|---|---|---|---|---|---|---|---|---|---|---|---|---|---|
| **Strain** | GEN | KAN | STP | NEO | TET | ERY | CLI | CHL | AMP | PEN | VAN | VIR | LIN | TRM | CIP | RIF |
| V3III-1 | 4 | 64 | 32 | 8 | 2 | 0.12 | 0.5 | 2 | 0.5 | 0.12 | >128 | 0.5 | 0.5 | 0.5 | 2 | 0.5 |
| V4II-90 | 4 | 256 | 32 | 8 | 2 | 0.12 | 0.5 | 2 | 0.5 | 0.12 | >128 | 0.5 | 1 | 0.25 | 4 | 1 |
| V7II-1 | 4 | 128 | 32 | 4 | 2 | 0.12 | 0.5 | 4 | 0.5 | 0.12 | >128 | 0.5 | 0.5 | 0.5 | 2 | 0.25 |
| V7II-62 | 2 | 128 | 32 | 8 | 2 | 0.25 | 0.5 | 2 | 0.5 | 0.25 | >128 | 0.25 | 1 | 0.25 | 2 | 0.5 |
| V7IV-1 | 8 | 256 | 32 | 4 | 2 | 0.12 | 0.5 | 2 | 0.5 | 0.25 | >128 | 0.5 | 1 | 0.5 | 2 | 0.5 |
| V7IV-60 | 8 | 128 | 32 | 8 | 2 | 0.12 | 0.4 | 4 | 0.5 | 0.25 | >128 | 0.5 | 1 | 0.5 | 2 | 0.5 |
| V8III-62 | 8 | 128 | 32 | 2 | 2 | 0.25 | 0.5 | 4 | 0.5 | 0.25 | >128 | 1 | 1 | 0.5 | 2 | 0.5 |
| V11I-60 | 4 | 128 | 32 | 8 | 2 | 0.12 | 0.5 | 2 | 0.5 | 0.25 | >128 | 1 | 1 | 0.5 | 2 | 0.5 |
| V11III-60 | 8 | 256 | 32 | 4 | 2 | 0.12 | 0.5 | 2 | 0.5 | 0.25 | >128 | 0.5 | 1 | 0.5 | 2 | 0.5 |
| V11IV-60 | 4 | 128 | 32 | 8 | 2 | 0.12 | 0.5 | 2 | 0.5 | 0.25 | >128 | 1 | 1 | 0.5 | 2 | 0.5 |
| Breakpoint^{b} | 16 | R | 64 | nr | 8 | 1 | 1 | 4 | 4 | nr | nr (R) | nr | nr | nr | nr | nr |

| | | | | | | | | | | | | | | | | |
|---|---|---|---|---|---|---|---|---|---|---|---|---|---|---|---|---|
| ^{a}Abbreviations: GEN, gentamycin; KAN, kanamycin; STP, streptomycin; NEO, neomycin; TET, tetracycline; ERY, erythromycin; CLI, clindamycin; CHL, chloramphenicol; AMP, ampicillin; PEN, penicillin; VAN, vancomycin; VIR, virginiamycin; LIN, linezolid; TRM, trimethoprim; CIP, ciprofloxacin; RIF, rifampicin; nr, not required by EFSA. R, the species L. salivarius is intrinsically resistant. ^{b}Breakpoint: microbiological breakpoints (mg /ml) that categorise Lactobacillus salivarius as resistant (microbiological breakpoints are defined as the MIC values that clearly deviate from those displayed by the normal susceptible populations; EFSA, 2012). | | | | | | | | | | | | | | | | |

**Table 8. Haematological parameters in rats after the 2-week observation period following a single oral dose of L. salivarius V4II-90 at 10¹⁰ cfu.**

| | **Group 1 (control)** | | **Group 2 (probiotic)** | |
|---|---|---|---|---|
| **Parameters^{a}** | Female | Male | Female | Male |
| RBC (x10⁶/ml) | 8.33 ± 0.31 | 8.34 ± 0.25 | 8.34 ± 0.55 | 8.35 ± 0.47 |
| Haemoglobin (g/dl) | 14.69 ± 0.49 | 14.87 ± 0.47 | 14.73 ± 0.54 | 14.85 ± 0.61 |
| Haematocrit (%) | 47.47 ± 0.60 | 47.64 ± 0.59 | 47.43 ± 0.69 | 47.69 ± 0.64 |
| MCV (fl) | 57.33 ± 1.06 | 58.43 ± 0.92 | 57.03 ± 0.87 | 58.49 ± 1.29 |
| MCH (pg) | 17.65 ± 0.57 | 17.75 ± 0.64 | 17.70 ± 0.55 | 17.74 ± 0.67 |
| MCHC (g/dl) | 31.63 ± 0.54 | 31.82 ± 0.43 | 31.59 ± 0.49 | 31.84 ± 0.43 |
| RDW (%) | 15.70 ± 0.42 | 15.78 ± 0.44 | 15.65 ± 0.56 | 15.82 ± 0.50 |
| WBC (×10³/ml) | 9.64 ± 0.72 | 9.90 ± 0.84 | 9.58 ± 0.65 | 9.93 ± 0.83 |
| Banded neutrophils (×10³/ml) | 0.00 ± 0.00 | 0.00 ± 0.00 | 0.00 ± 0.00 | 0.00 ± 0.00 |
| Neutrophils (x103/ml) | 1.47 ± 0.20 | 1.49 ± 0.15 | 1.46 ± 0.17 | 1.50 ± 0.26 |
| Eosinophils (×10³/ml) | 0.04 ± 0.01 | 0.05 ± 0.01 | 0.05 ± 0.01 | 0.04 ± 0.01 |
| Lymphocytes (×10³/ml) | 7.02 ± 0.62 | 9.01 ± 0.52 | 7.11 ± 0.56 | 9.03 ± 0.60 |
| Monocytes (×10³/ml) | 0.32 ± 0.07 | 0.35 ± 0.08 | 0.29 ± 0.07 | 0.34 ± 0.07 |
| Basophils (x103/ml) | 0.00 ± 0.00 | 0.00 ± 0.00 | 0.00 ± 0.00 | 0.00 ± 0.00 |
| Platelets (×10³/ml) | 713.10 ± 35.23 | 729.69 ± 29.24 | 716.76 ± 38.12 | 731.61 ± 33.33 |
| MPV (fl) | 6.31 ± 0.14 | 6.80 ± 0.15 | 6.33 ± 0.13 | 6.80 ± 0.11 |

| | | | | |
|---|---|---|---|---|
| ^{a}Data are expressed as mean ± SEM (n = 6 animals/sex group). MCV, mean corpuscular volume; MCH, mean corpuscular hemoglobin; MCHC, mean corpuscular hemoglobin concentration; RDW, red cell distribution width; WBC, white blood cell; MPV, mean platelet volume. | | | | |

**Table 9. Clinical chemistry parameters in rats after the 2-week observation period following a single oral dose of L. salivarius V4II-90 at 10¹⁰ cfu.**

| | **Group 1 (control)** | | **Group 2 (probiotic)** | |
|---|---|---|---|---|
| **Parameters^{a}** | Female | Male | Female | Male |
| Glucose (mg/dl) | 119.02 ± 4.27 | 122.21 ± 4.42 | 119.92 ± 4.28 | 121.47 ± 5.32 |
| Urea nitrogen (mg/dl) | 48.67 ± 4.09 | 44.32 ± 3.76 | 49.33 ± 3.55 | 44.07 ± 4.53 |
| Creatinine (mg/dl) | 0.60 ± 0.07 | 0.53 ± 0.09 | 0.59 ± 0.07 | 0.52 ± 0.06 |
| Total protein (g/dl) | 7.53 ± 0.42 | 6.84 ± 0.34 | 7.51 ± 0.43 | 6.86 ± 0.41 |
| Total bilirubin (mg/dl) | 0.17 ± 0.03 | 0.18 ± 0.05 | 0.17 ± 0.04 | 0.18 ± 0.04 |
| Calcium (mg/dl) | 11.92 ± 0.56 | 11.84 ± 0.46 | 11.88 ± 0.44 | 11.85 ± 0.35 |
| Sodium (mEq/l) | 145.32 ± 6.04 | 148.40 ± 7.42 | 144.10 ± 6.52 | 149.24 ± 7.07 |
| Potassium (mEq/l) | 5.88 ± 0.47 | 6.42 ± 0.62 | 5.82 ± 0.51 | 6.45 ± 0.57 |
| ASAT (u/l) | 150.21 ± 7.84 | 157.44 ± 8.56 | 151.74 ± 8.14 | 157.58 ± 7.69 |
| ALAT (u/l) | 62.75 ± 5.82 | 70.19 ± 6.21 | 61.96 ± 5.65 | 70.72 ± 6.13 |
| Alkaline phosphatase (u/l) | 277.31 ± 35.25 | 823.85 ± 39.69 | 270.88 ± 37.46 | 820.36 ± 40.21 |
| Triglyceride (mg/dl) | 107.14 ± 8.46 | 128.10 ± 8.31 | 104.11 ± 9.31 | 126.56 ± 9.24 |
| Cholesterol (mg/dl) | 66.21 ± 5.47 | 62.21 ± 5.69 | 66.05 ± 6.24 | 62.46 ± 5.92 |
| HDL (mg/dl) | 47.33 ± 3.72 | 42.08 ± 4.01 | 46.82 ± 4.05 | 42.31 ± 4.02 |
| LDL (mg/dl) | 7.67 ± 0.76 | 8.46 ± 0.82 | 7.65 ± 0.79 | 8.44 ± 0.69 |

| | | | | |
|---|---|---|---|---|
| ^{a}Data are expressed as mean ± SEM (n = 6 animals/sex group). | | | | |

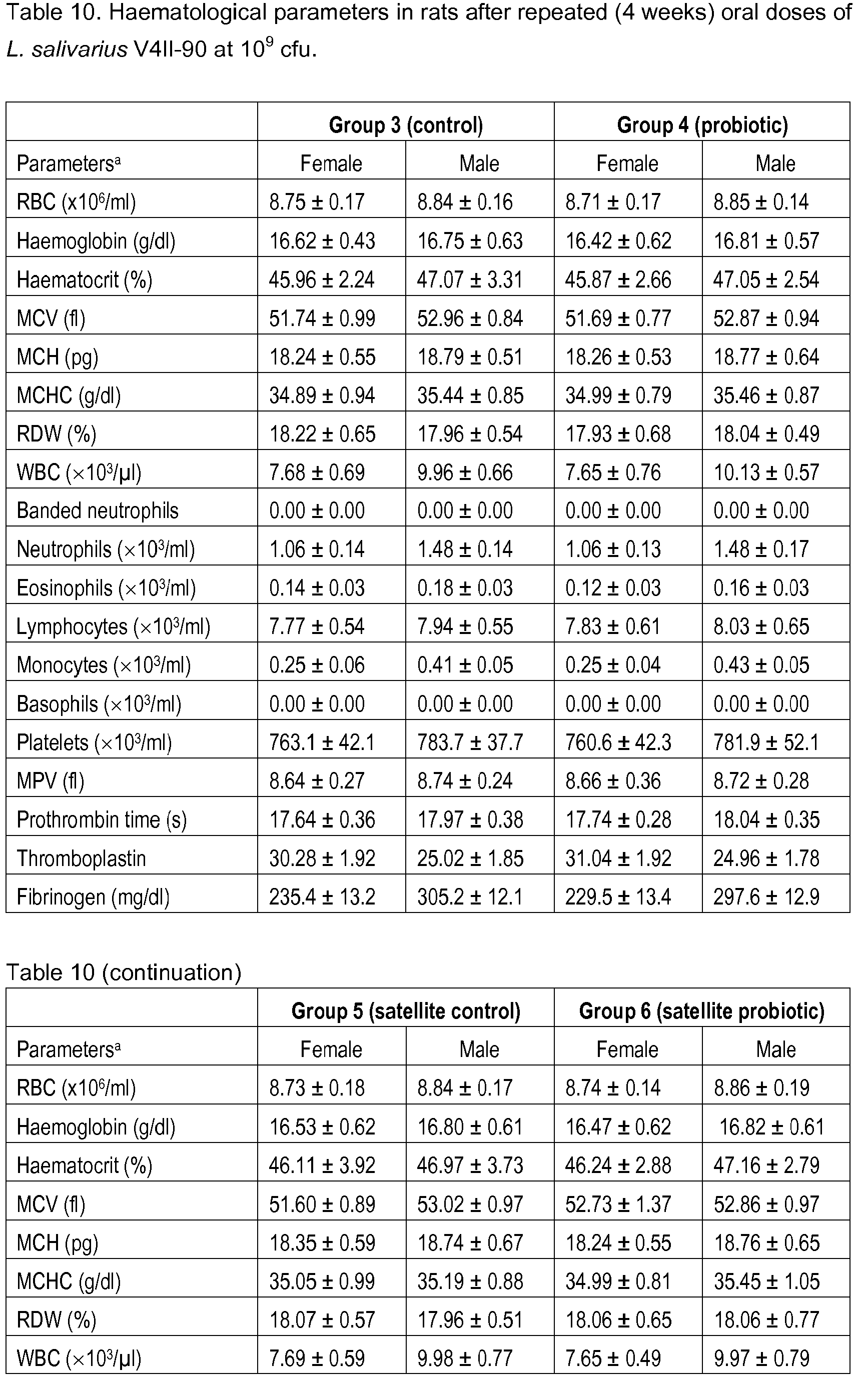

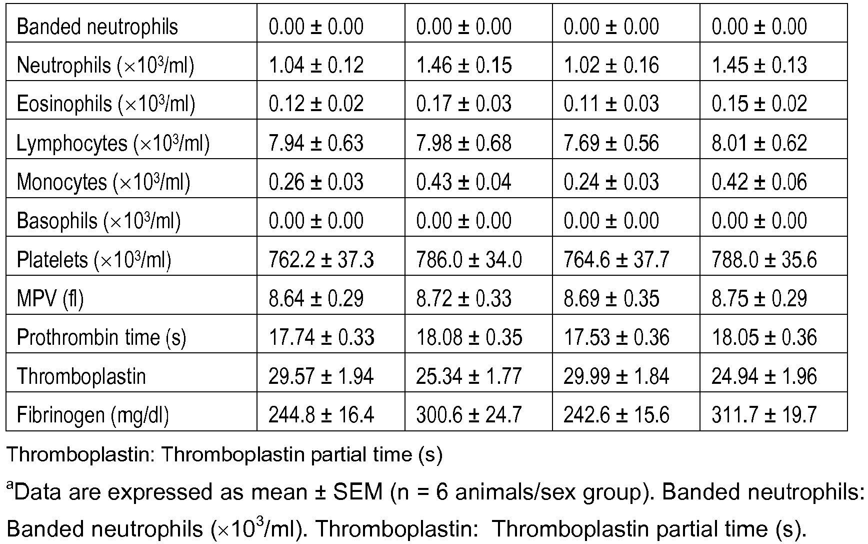

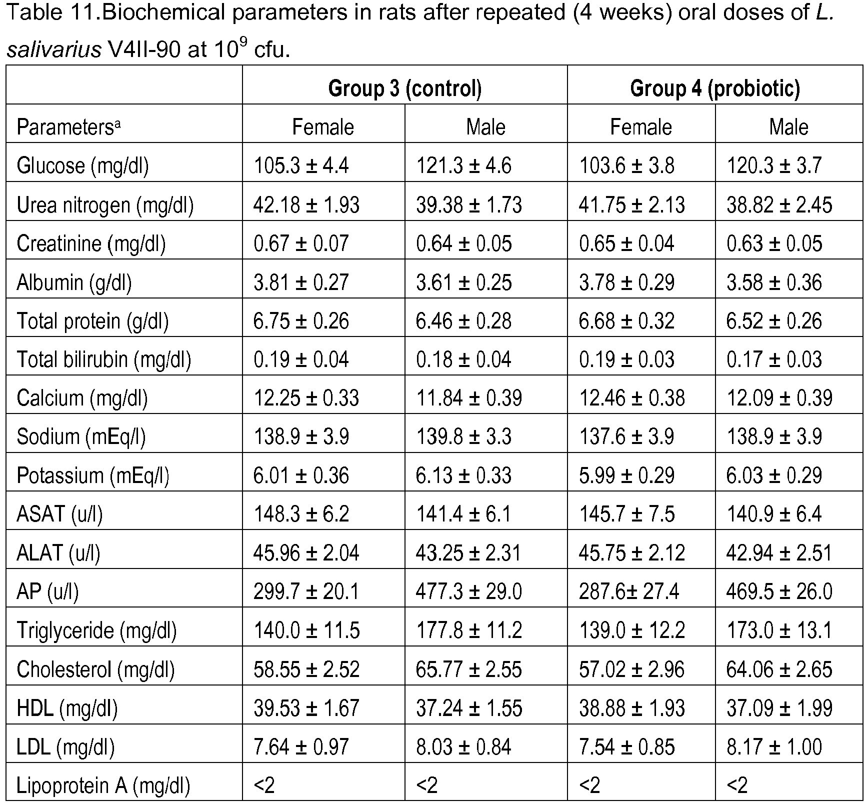

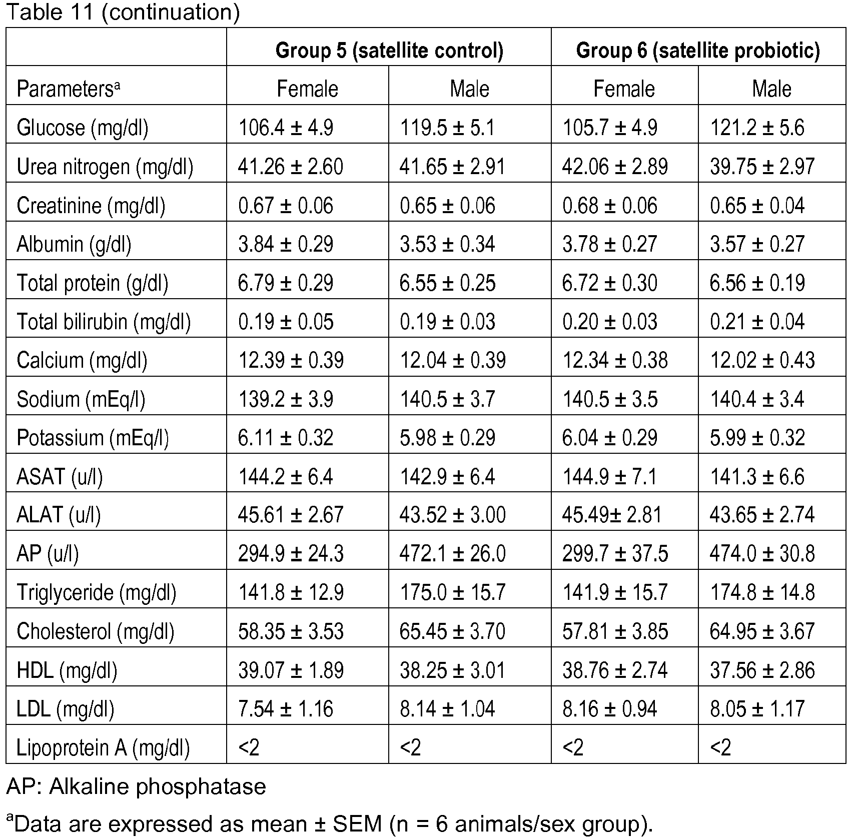

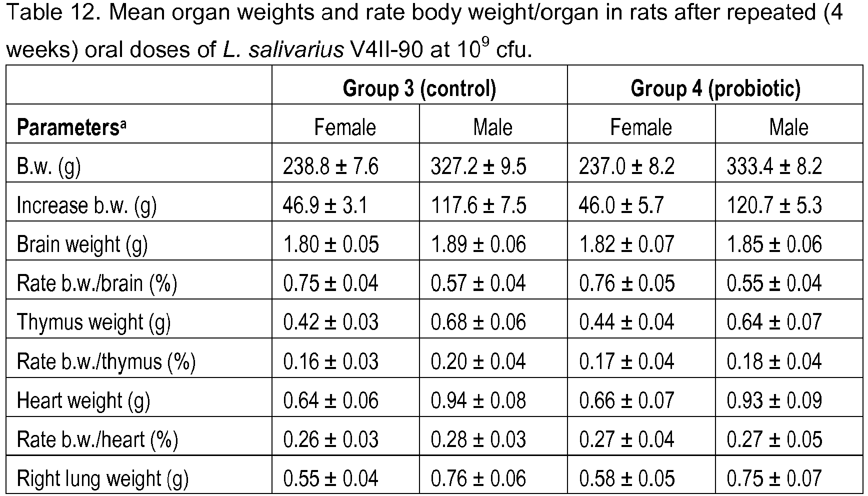

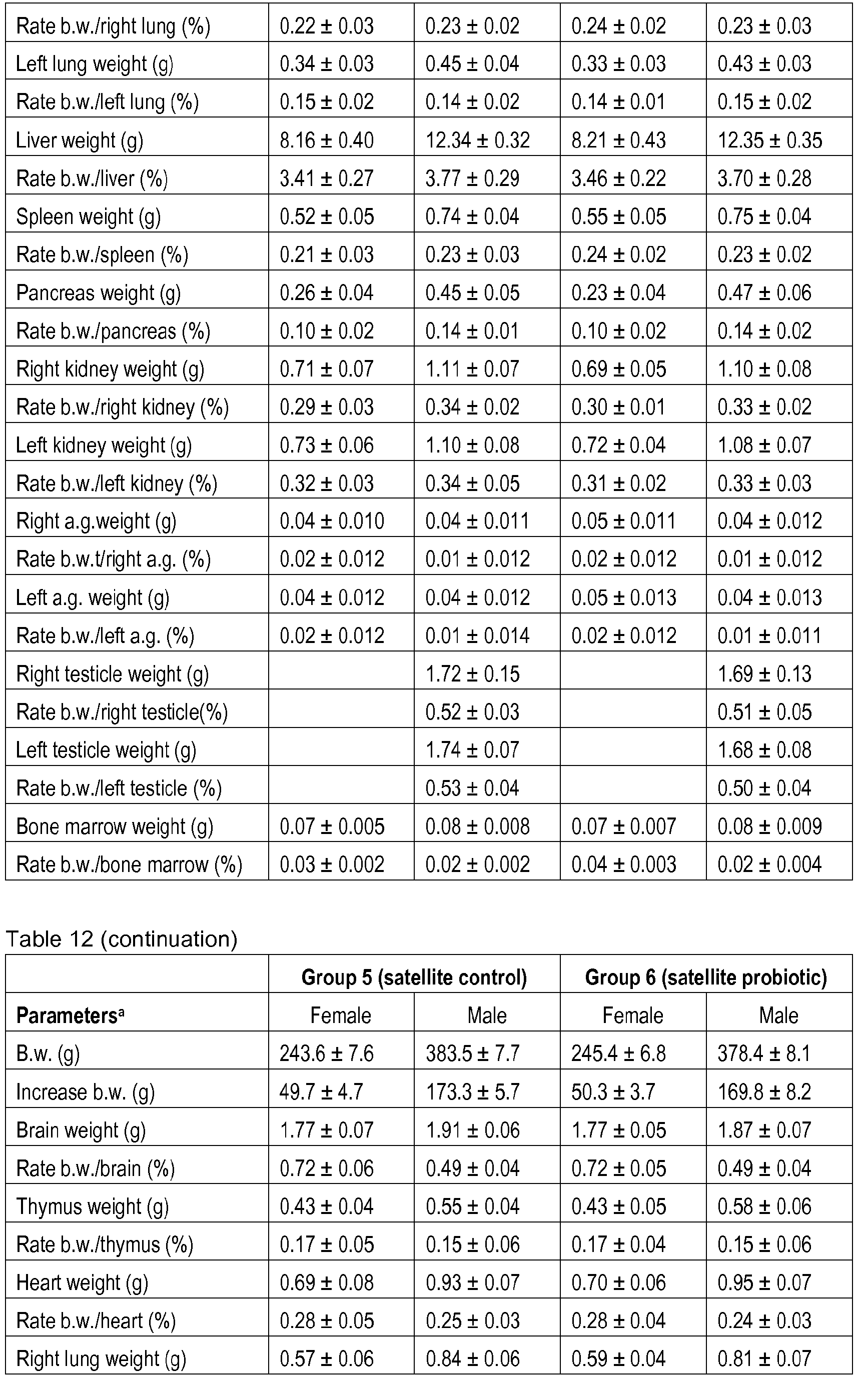

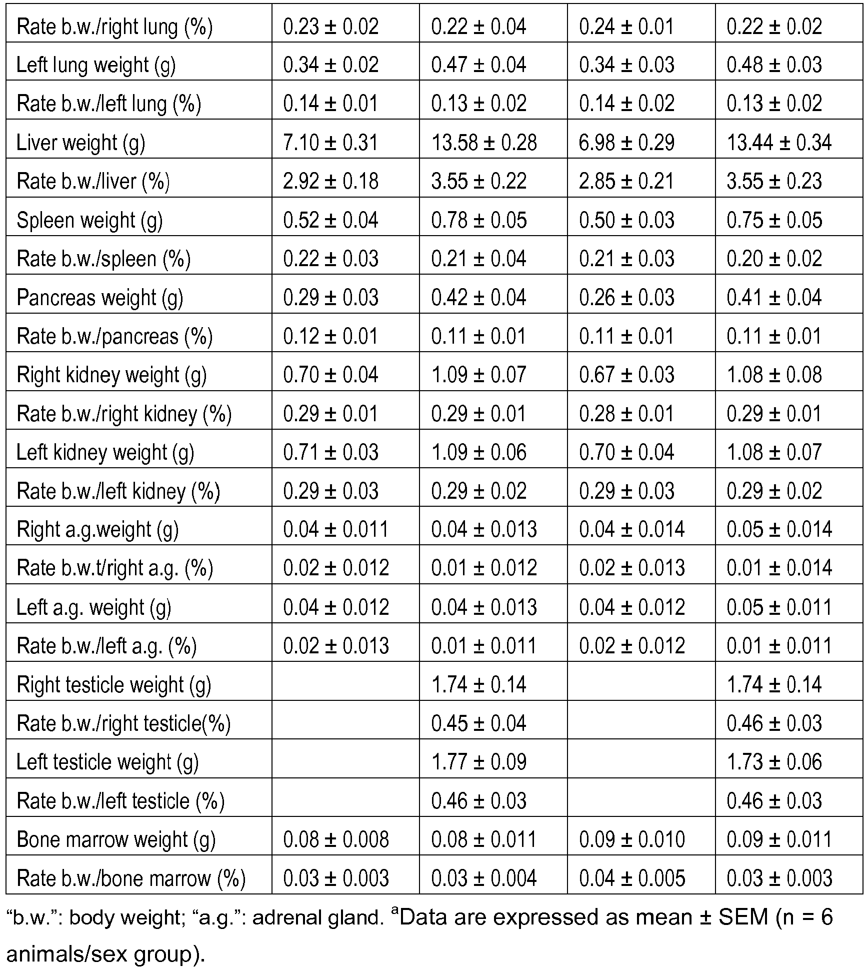

**Table 13. Specificity of the real-time PCR system (Ct values obtained from 10 ng DNA).**

| **Scientific name** | **Crossing point (Ct)** | **Melting temperature (Tm)** |
|---|---|---|
| *Streptococcus agalactiae* DSM 2134 | 16.77±1.51*^{a}* | 80.00 |
| *Streptococcus agalactiae* M57207 | 18.08±1.14 | 80.00 |
| *Streptococcus agalactiae* M57730 | 17.97±0.58 | 80.00 |
| *Streptococcus agalactiae* M67018 | 16.72±0.45 | 80.00 |
| *Streptococcus agalactiae* M6836 | 19.07±0.81 | 80.00 |
| *Streptococcus agalactiae* M70043 | 20.87±0.31 | 80.00 |
| *Streptococcus dysgalactiae* DSM 20662 | 40.00±0.00 | None |
| *Streptococcus peroris* DSM 12493 | 40.00±0.00 | None |
| *Streptococcus mitis* DSM 12643 | 39.01±0.54 | None |
| *Streptococcus oralis* CECT 907 | 40.00±0.00 | None |
| *Streptococcus parasanguinis* DSM 6778 | 40.00±0.00 | None |
| *Streptococcus bovis* DSM 20564 | 38.73±0.51 | None |
| *Streptococcus uberis* DSM 20564 | 40.00±0.00 | None |
| *Streptococcus salivarius* CECT 805 | 39.83±0.10 | None |
| *Streptococcus termophilus* ATCC 19987 | 40.00±0.00 | None |
| *Streptococcus pneunoniae* 0566 | 40.00±0.00 | None |
| *Bifidobacterium adolescentis* HE 005 | 40.00±0.00 | None |
| *Bifidobacterium animalis* subsp. *lactis PS17* | 40.00±0.00 | None |
| *Bifidobacterium breve* CECT 4839 | 40.00±0.00 | None |
| *Bifidobacterium infantis* CECT 4551 | 40.00±0.00 | None |
| *Bacteroides vulgatus* DSM 1447 | 40.00±0.00 | None |
| *Bacteroides fragilis* DSM 2151 | 40.00±0.00 | None |
| *Leuconostoc citreum* CECT 4025 | 40.00±0.00 | None |
| *Leuconostoc mesenteroides* CECT 219 | 40.00±0.00 | None |
| *Leuconostoc fallax* LMG 13177 | 40.00±0.00 | None |
| *Weissella cibaria* CECT 7032 | 40.00±0.00 | None |
| *Weissella confusa* CECT 4707 | 40.00±0.00 | None |
| *Lactobacillus crispatus* DSMZ 20584 | 40.00±0.00 | None |
| *Lactobacillus delbrueckii subsp. lactis* CECT 282 | 37.09±1.23 | None |
| *Lactobacillus salivarius* CECT 4063 | 40.00±0.00 | None |
| *Staphylococcus epidermidis* CECT 232 | 40.00±0.00 | None |
| *Staphylococcus aureus* CECT 86 | 40.00±0.00 | None |
| *Lactococcus lactis* ATCC 10456 | 40.00±0.00 | None |
| *Pediococcus pentosaceus* CECT 46595 | 40.00±0.00 | None |
| *Klebsiella pneumoniae subsp. Pneumoniae* DSM 30104 | 40.00±0.00 | None |
| *Enterococcus faecium* CECT 410 | 40.00±0.00 | None |
| *Enterococcus faecalis* CECT 481 | 40.00±0.00 | None |

| | | |
|---|---|---|
| *^{a}*Ct values are expressed as mean ± standard deviation | | |

**Table 14. Detection limit of the real-time PCT system using ten-fold series of S. agalactiae DMSZ 2134 DNA as standard.**

| **Dilution** | **Ct value** | **DNA concentration (ng)** |
|---|---|---|
| 10⁻¹ | 19.24±0.02 | 110 |
| 10⁻² | 20.09±0.15 | 55 |
| 10⁻³ | 20.22±0.23 | 27.5 |
| 10⁻⁴ | 21.80±0.07 | 13.75 |
| 10⁻⁵ | 22.64±0.04 | 6.8 |
| 10⁻⁶ | 23.48±0.11 | 3.43 |
| 10⁻⁷ | 24.83±0.07 | 1.71 |
| 10⁻⁸ | 25.66±0.12 | 0.85 |
| 10⁻⁹ | 26.52±0.01 | 0.42 |
| 10⁻¹⁰ | 27.66±0.04 | 0.21 |
| 10⁻¹¹ | 29.82±0.13 | 0.1 |
| 10⁻¹² | 30.37±0.00 | 0.05 |
| 10⁻¹³ | 31.61±0.10 | 0.02 |
| 10⁻¹⁴ | 33.18±0.15 | 0.01 |
| 10⁻¹⁵ | 34.34±0.21 | 0.005 |

**Table 15. Results of conventional and real-time PCR assay to the detection of GBS vaginally-colonized women.**

| **Sample** | **Culture and identification by MALDI-TOF** | **Conventional PCR amplification results (bp)** | **Real-time PCR results (Ct)** |
|---|---|---|---|
| 1 | + | 148 bp | 21.82±0.50 *^{a}* |
| 2 | + | 148 bp | 33.81±0.74 |
| 3 | - | - | 40±0.00 |
| 4 | + | 148 bp | 20.85±0.53 |
| 5 | - | - | 40±0.00 |
| 6 | - | - | 40±0.00 |
| 7 | - | - | 40±0.00 |
| 8 | - | - | 40±0.00 |
| 9 | - | - | 40±0.00 |
| 10 | + | 148 bp | 22.03±0.53 |
| 11 | - | - | 40±0.00 |
| 12 | - | - | 40±0.00 |
| 13 | - | - | 40±0.00 |
| 14 | - | - | 40±0.00 |
| 15 | - | - | 40±0.00 |
| 16 | - | - | 40±0.00 |
| 17 | - | - | 40±0.00 |
| 18 | + | 148 bp | 32.28±0.30 |
| 19 | - | - | 40±0.00 |
| 20 | - | - | 40±0.00 |
| 21 | - | - | 40±0.00 |
| 22 | - | - | 40±0.00 |
| 23 | - | - | 40±0.00 |
| 24 | - | - | 40±0.00 |
| 25 | + | 148 bp | 22.01±1.3 |
| 26 | - | - | 40±0.00 |
| 27 | - | 148 bp | 35.98±0.97 |
| 28 | + | 148 bp | 40±0.00 |
| 29 | + | 148 bp | 20.82±1.26 |
| 30 | - | - | 40±0.00 |
| 31 | - | - | 40±0.00 |
| 32 | - | - | 40±0.00 |
| 33 | - | - | 40±0.00 |
| 34 | + | 148 bp | 25.30±0.49 |
| 35 | - | - | 40±0.00 |
| 36 | - | - | 40±0.00 |
| 37 | + | 148 bp | 18.99±0.31 |
| 38 | + | 148 bp | 40±0.00 |
| 39 | - | - | 40±0.00 |
| 40 | - | - | 40±0.00 |
| 41 | - | - | 40±0.00 |
| 42 | - | - | 40±0.00 |
| 43 | + | 148 bp | 34.40±1.09 |
| 44 | + | 148 bp | 33.01±1.74 |
| 45 | - | - | 40±0.00 |
| 46 | - | - | 40±0.00 |
| 47 | + | 148 bp | 30.12±1.66 |
| 48 | + | 148 bp | 29.35±0.42 |
| 49 | - | - | 40±0.00 |
| 50 | - | - | 40±0.00 |
| 51 | + | 148 bp | 30.16±1.22 |
| 52 | - | - | 40±0.00 |
| 53 | + | 148 bp | 24.99±1.69 |
| 54 | - | - | 40±0.00 |
| 55 | - | - | 40±0.00 |
| 56 | + | 148 bp | 29.98±1.10 |
| 57 | + | 148 bp | 33.70±1.17 |
| 58 | - | - | 40±0.00 |
| 59 | - | - | 40±0.00 |
| 60 | - | - | 40±0.00 |
| 61 | + | 148 bp | 27.54±1.00 |
| 62 | - | - | 40±0.00 |

| | | | |
|---|---|---|---|
| + Positive GBS isolation; -Negative GBS isolation/GBS DNA detection. *^{a}*Ct values are expressed as mean ± standard deviation. | | | |

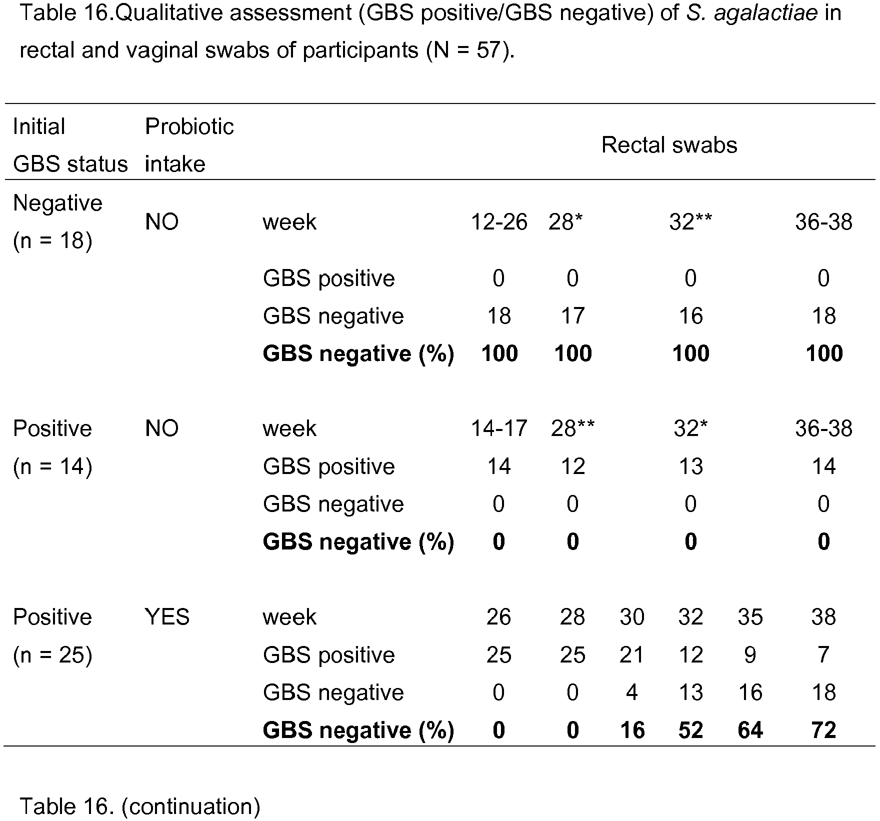

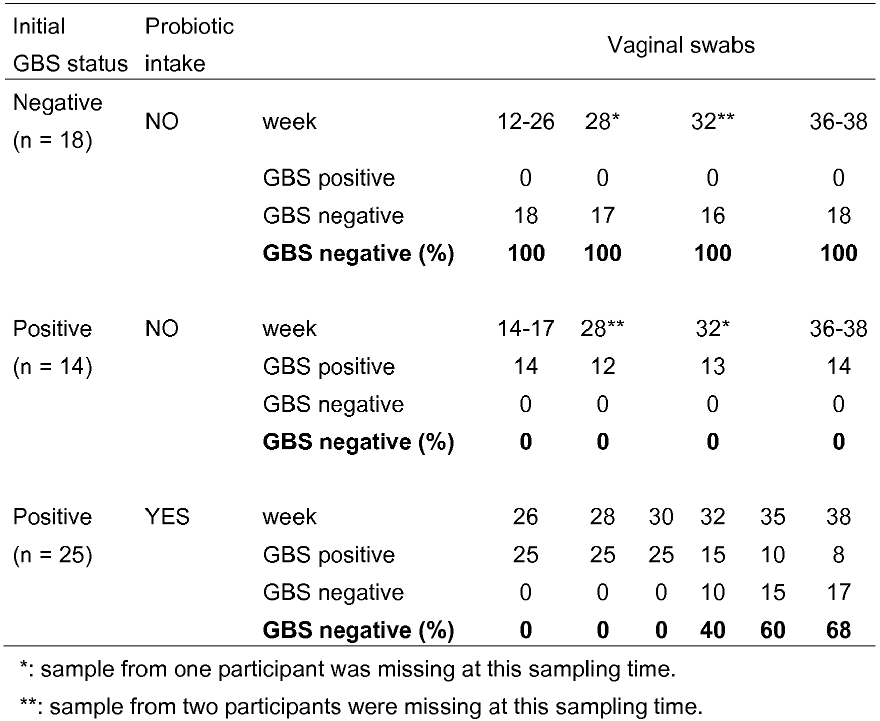

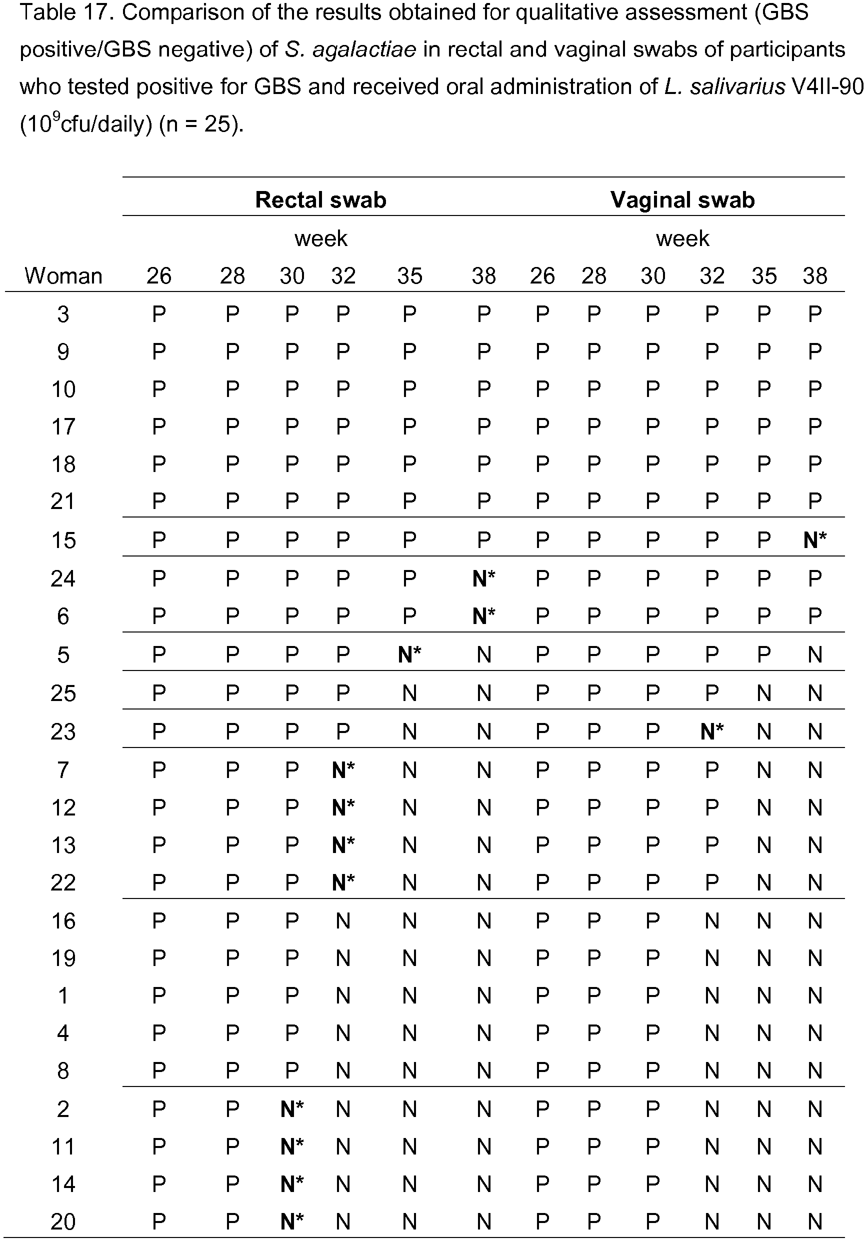

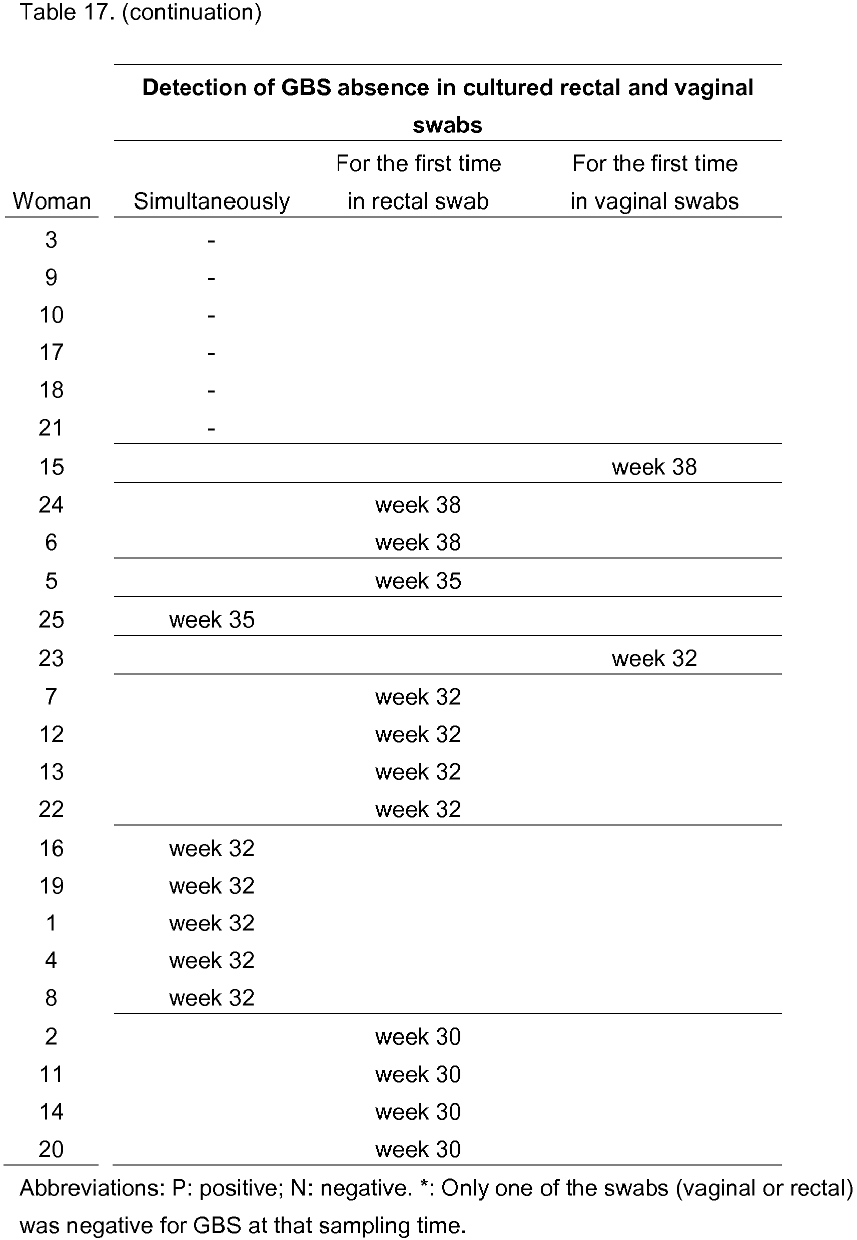

### REFERENCES CITED IN THE APPLICATION:

Arroyo R, *et al.* 2010 Clin Infect Dis 50:1551-1558.
Boris S, et al. 1998 Infect Immun. 66:1985-1989.
Bover-Cid S and Holzapfel WH 1999 International Journal of Food Microbiology 53:33-41
Coconnier MH, et al. 1992 Appl Environ Microbiol 58:2034-2039.
Conway PL, et al. 1987 Journal of Dairy Science 70:1-12.
De Gregorio PR, et al. 2014 J Med Microbiol 63:685-696.
EFSA. Panel on additives and products or substances used in animal feed (FEEDAP). Guidance on the assessment of bacterial susceptibility to antimicrobials of human and veterinary importance. EFSA Journal. 2012. 10, 2740.
Garcia-Moruno E, et al. 2005 J Food Protect 68(3):625-9.
Kullen, M J, et al. 2000 J. Appl. Microbiol 89: 511-516.
Ladero V, et al. 2012 Food Microbiol 30(1):132-8.
Langa S, et al. 2012 Appl Microbiol Biotechnol. Jun;94(5):1279-87.
Le Jeune C, et al. 1995 J Appl Bacteriol;78:316-26.
Lucas P and Lonvaud-funel A. 2002 FEMS Microbiol Lett 211:85-9.
Magnusson J and Schnürer J. 2001 Applied and Environmental Microbiology 67:1-5.
Marteau P, et al. 1997 Journal of Dairy Science 80:1031-1037.
Martin R, et al. 2005 J Hum Lact; 21: 8-17.
Martin R, et al. 2006 Int J Food Microbiol 112:35-43.
Martin R, et al. 2009 J Dairy Res. Nov 76(4):418-25.
Muller AE, et al. 2006 Acta Obstet Gynecol Scand 85:1027-1037.
Reid G, et al. 1990 Current Microbiology 20: 47-52.
Ruiz-Barba JL, et al. 2005 Anal Biochem 347:333-5
Savini V, et al. 2013 Int J Clin Exp Pathol 6:1693-1695.
Song Y-L, et al. 1999 J Clin Microbiol 37:3062-3064.
van den Hoogen A, et al. 2010 Neonatology 97:22-8.
Yap PS and Gilliland SE 2000 J. Dairy Sci 83:628-632.
Zhou JS, et al. 2001 International Journal of Food Microbiology 63:81-90.

## Claims

1. An isolated strain of *Lactobacillus salivarius* deposited at the "Colección Española de Cultivos Tipo (CECT)" under the accession number CECT 9145.

2. A bacterial culture which comprises the strain of claim 1.

3. The isolated strain according to claim 1 or the bacterial culture according to claim 2 for use as a medicament.

4. The isolated strain according to claim 1 or the bacterial culture according to claim 2 for use in the prevention and/or treatment of an infection disease.

5. The isolated strain or the bacterial culture for use according to claim 4 wherein the infection disease is caused by *Streptococcus agalactiae.*

6. The isolated strain or the bacterial culture for use according to claim 5 wherein the infection disease caused by *Streptococcus agalactiae* is early onset sepsis, a gynecological infection or mastitis.

7. The isolated strain according to claim 1 or the bacterial culture according to claim 2 for use as a probiotic.

8. A pharmaceutical composition comprising a therapeutically effective amount of the strain according to claim 1 or the bacterial culture according to claim 2 as active ingredient, together with appropriate amounts of a pharmaceutical acceptable excipient and/or carrier.

9. The pharmaceutical composition according to claim 8 which is for oral, vaginal, rectal, anal, intramammary, intravenous, intraarterial, parenteral, intracranial, topical, respiratory or nasal administration.

10. An edible product which comprises an effective amount of the strain according to claim 1 or the bacterial culture according to claim 2 as active ingredient, together with appropriate amount of another edible ingredient.

11. The edible product according to claim 10, which is selected from the group consisting of a milk product, a yogurt, a curd, a cheese, a fermented milk, a powdered milk, a milk based fermented product, a meat based fermented product, an ice-cream, a cereal based fermented product, a beverage, a snack, a flour, a chewing-gum, a sweet, a sweet food, a pet food, a dietary or food supplement, a functional food, a nutraceutical, a clinical nutrition formula, a nutritional complement, a formula for pregnant woman, a formula for the elderly and an infant formula.

12. The edible product according to any one of claims 10 or 11, wherein the effective amount (dose) of the strain as defined in claim 1 or of the bacterial culture as defined in claim 2 is comprised from 10⁸cfu/dose to 10¹⁰cfu/dose of the edible product.

13. A medical device comprising an effective amount of the strain according to claim 1 or of the bacterial culture according to claim 2 as an active ingredient.

14. A method to obtain a mutant of the strain of *Lactobacillus salivarius* CECT 9145, comprising using the deposited strain as starting material and applying mutagenesis, wherein the obtained mutant retains or enhances the activity of the parent deposited strain to prevent or treat diseases caused by S. *agalactiae.*

15. A kit that comprises an effective amount of the strain according to claim 1, the bacterial culture according to claim 2 or the pharmaceutical composition according to any one of claims 8 or 9.

## Patentansprüche

1. Ein isolierter Stamm von *Lactobacillus salivarius,* der bei der "Colección Española de Cultivos Tipo (CECT)" unter der Hinterlegungsnummer CECT 9145 hinterlegt ist.

2. Eine Bakterienkultur, welche den Stamm des Anspruchs 1 umfasst.

3. Der isolierter Stamm nach Anspruch 1 oder die Bakterienkultur nach Anspruch 2 zur Verwendung als Arzneimittel.

4. Der isolierter Stamm nach Anspruch 1 oder die Bakterienkultur nach Anspruch 2 zur Verwendung in der Prävention und/oder Behandlung einer Infektionskrankheit.

5. Der isolierter Stamm oder die Bakterienkultur zur Verwendung nach Anspruch 4, wobei die Infektionskrankheit durch *Streptococcus agalactiae* verursacht wird.

6. Der isolierter Stamm oder die Bakterienkultur zur Verwendung nach Anspruch 5, wobei die durch *Streptococcus agalactiae* verursachte Infektionskrankheit früh einsetzende Sepsis, eine gynäkologische Infektion oder Mastitis ist.

7. Der isolierter Stamm nach Anspruch 1 oder die Bakterienkultur nach Anspruch 2 zur Verwendung als Probiotikum.

8. Eine pharmazeutische Zusammensetzung umfassend eine therapeutisch wirksame Menge des Stammes nach Anspruch 1 oder der Bakterienkultur nach Anspruch 2 als Wirkstoff, zusammen mit geeigneten Mengen eines pharmazeutisch akzeptablen Hilfsstoffs und/oder Trägers.

9. Die pharmazeutische Zusammensetzung nach Anspruch 8, die zur oralen, vaginalen, rektalen, analen, intramammären, intravenösen, intraarteriellen, parenteralen, intrakraniellen, topischen, respiratorischen oder nasalen Verabreichung bestimmt ist.

10. Ein essbares Produkt, das eine wirksame Menge des Stammes nach Anspruch 1 oder die Bakterienkultur nach Anspruch 2 als Wirkstoff, zusammen mit einer geeigneten Menge eines anderen essbaren Bestandteils, umfasst.

11. Das essbare Produkt nach Anspruch 10, welches ausgewählt ist aus der Gruppe bestehend aus einem Milchprodukt, einem Joghurt, einem Quark, einem Käse, einer fermentierten Milch, einem Milchpulver, einem fermentierten Produkt auf Milchbasis, einem fermentierten Produkt auf Fleischbasis, einem Eis, einem fermentierten Produkt auf Getreidebasis, einem Getränk, einem Snack, einem Mehl, einem Kaugummi, einer Süßigkeit, einem süßen Lebensmittel, einem Tierfutter, einem Nahrung- oder Ernährungsergänzungsmittel, einem funktionellen Lebensmittel, einem Nutrazeutikum, einer klinischen Ernährungsrezeptur, einer Nahrungsergänzung, einer Nahrung für schwangere Frauen, einer Nahrung für ältere Menschen und eine Säuglingsnahrung.

12. Das essbare Produkt nach einem der Ansprüche 10 oder 11, wobei die wirksame Menge (Dosis) des Stammes wie in Anspruch 1 definiert oder der Bakterienkultur wie in Anspruch 2 definiert von 10⁸ KBE/Dosis bis 10¹⁰ KBE/Dosis des essbaren Produkts reicht.

13. Eine medizinische Vorrichtung umfassend eine wirksame Menge des Stammes nach Anspruch 1 oder der Bakterienkultur nach Anspruch 2 als Wirkstoff.

14. Ein Verfahren zum Erhalten einer Mutante des Stammes von *Lactobacillus salivarius* CECT 9145, umfassend das Verwenden des hinterlegten Stammes als Ausgangsmaterial und das Anwenden von Mutagenese, wobei die erhaltene Mutante die Aktivität des hinterlegten Elternstamms beibehält oder verstärkt, um durch S. *agalactiae* verursachte Krankheiten zu verhindern oder zu behandeln.

15. Ein Kit, das eine wirksame Menge des Stammes nach Anspruch 1, der Bakterienkultur nach Anspruch 2 oder der pharmazeutischen Zusammensetzung nach einem der Ansprüche 8 oder 9 umfasst.

## Revendications

1. Une souche isolée de *Lactobacillus salivarius* déposée à la « Colección Española de Cultivos Tipo (CECT) » sous le numéro de dépôt CECT 9145.

2. Une culture bactérienne qui comprend la souche de la revendication 1.

3. La souche isolée selon la revendication 1 ou la culture bactérienne selon la revendication 2 pour l'utilisation comme médicament.

4. La souche isolée selon la revendication 1 ou la culture bactérienne selon la revendication 2 pour l'utilisation dans la prévention et / ou le traitement d'une maladie infectieuse.

5. La souche isolée ou la culture bactérienne pour l'utilisation selon la revendication 4, dans laquelle la maladie infectieuse est provoquée par *Streptococcus agalactiae.*

6. La souche isolée ou la culture bactérienne pour l'utilisation selon la revendication 5, dans laquelle la maladie infectieuse provoquée par *Streptococcus agalactiae* est une septicémie à début précoce, une infection gynécologique ou une mammite.

7. La souche isolée selon la revendication 1 ou la culture bactérienne selon la revendication 2 pour l'utilisation comme probiotique.

8. Une composition pharmaceutique comprenant une quantité thérapeutiquement efficace de la souche selon la revendication 1 ou de la culture bactérienne selon la revendication 2 comme ingrédient actif, conjointement avec des quantités appropriées d'un excipient et / ou véhicule pharmaceutiquement acceptable.

9. La composition pharmaceutique selon la revendication 8, qui est destinée à une administration orale, vaginale, rectale, anale, intramammaire, intraveineuse, intraartérielle, parentérale, intracrânienne, topique, respiratoire ou nasale.

10. Un produit comestible qui comprend une quantité efficace de la souche selon la revendication 1 ou de la culture bactérienne selon la revendication 2 comme ingrédient actif, conjointement avec une quantité appropriée d'un autre ingrédient comestible.

11. Le produit comestible selon la revendication 10, qui est choisi dans le groupe constitué d'un produit laitier, un yogourt, un caillé, un fromage, un lait fermenté, un lait en poudre, un produit fermenté à base de lait, un produit fermenté à base de viande, une crème glacée, un produit fermenté à base de céréales, une boisson, une collation, une farine, un chewing-gum, une sucrerie, un aliment sucré, un aliment pour animaux de compagnie, un complément diététique ou alimentaire, un aliment fonctionnel, un nutraceutique, une formule nutritionnelle clinique, un complément nutritionnel, une formule pour des femmes enceinte, une formule pour les personnes âgées et une préparation pour nourrissons.

12. Le produit comestible selon l'une quelconque des revendications 10 ou 11, dans lequel la quantité efficace (dose) de la souche telle que définie dans la revendication 1 ou de la culture bactérienne telle que définie dans la revendication 2 est comprise de 10⁸ ufc / dose à 10¹⁰ ufc / dose du produit comestible.

13. Un dispositif médical comprenant une quantité efficace de la souche selon la revendication 1 ou de la culture bactérienne selon la revendication 2 comme ingrédient actif.

14. Un procédé pour obtenir un mutant de la souche de *Lactobacillus salivarius* CECT 9145, comprenant l'utilisation de la souche déposée comme matière de départ et l'application de mutagenèse, dans lequel le mutant obtenu conserve ou améliore l'activité de la souche parentale déposée pour prévenir ou traiter des maladies causées par *S.agalactiae.*

15. Un kit qui comprend une quantité efficace de la souche selon la revendication 1, la culture bactérienne selon la revendication 2 ou la composition pharmaceutique selon l'une quelconque des revendications 8 ou 9.
